# EUROPEAN PATENT SPECIFICATION

(11) **EP 4 270 409 B1**
(45) Date of publication and mention of the grant of the patent: **16.09.2026**
(21) Application number: 21915909.2
(22) Date of filing: 23.12.2021
(51) Int. Cl.: G16H 20/70, G16H 10/60, G16H 50/20, G16H 50/30, A61B 5/00, A61N 2/02, A61N 2/00, A61M 21/00, A61B 5/055

(54) **MAGNETIC STIMULATION SYSTEM**
MAGNETISCHES STIMULATIONSSYSTEM
SYSTÈME DE STIMULATION MAGNÉTIQUE

(30) Priority: 28.12.2020 KR 20200185275; 28.12.2020 KR 20200185276
(43) Date of publication of application: 01.11.2023
(73) Proprietor: Advanced Technology & Communications Co., Ltd., Incheon 21635 (KR)
(72) Inventor: LEE, Jong-Won, Gunpo-si, Gyeonggi-do 15845 (KR); NA, Gi-Yong, Seoul 06375 (KR)
(74) Representative: Ström & Gulliksson AB
(86) International application number: PCT/KR2021/095132
(87) International publication number: WO 2022/146112

(56) References cited:
- JP-A- 2008 537 495
- KR-A- 20160 044 183
- KR-A- 20200 070 927
- KR-A- 20200 139 536
- KR-B1- 101 295 187
- KR-B1- 101 921 888
- US-A1- 2007 260 107
- US-A1- 2009 156 884
- US-A1- 2010 087 698
- GROPPA S ET AL: "A practical guide to diagnostic transcranial magnetic stimulation: Report of an IFCN committee", CLINICAL NEUROPHYSIOLOGY, ELSEVIER, AMSTERDAM, NL, vol. 123, no. 5, 22 January 2012 (2012-01-22), pages 858 - 882, XP028474459, ISSN: 1388-2457, [retrieved on 20120126], DOI: 10.1016/J.CLINPH.2012.01.010

## Description

### [Technical Field]

The present disclosure relates to a magnetic stimulation system

### [Background Art]

A magnetic stimulation method such as transcranial magnetic stimulation (TMS) may non-invasively stimulate nerve cells in the human brain by using magnetic energy. This method may activate the nerve cells by allowing a strong magnetic field to passes through the skull near the human head, and thus be used to perform a psychiatric treatment such as for depression or a neurological treatment such as for dementia.

Medical personnel such as nurses conventionally have to directly position a coil for the coil for generating a magnetic field to be positioned over a brain region that is to receive the magnetic stimulation. In this case, a lot of time has to be devoted to accurately positioning the coil, which may lower treatment efficiency.

In addition, cognitive training may be performed alternately with the transcranial magnetic stimulation method to maximize activation of the stimulation in the brain region. In particular, the cognitive training may be performed for a variety of reasons. However, the cognitive training may be used to improve or prevent cognitive function impairment, and may be performed for a user, for example, a patient with senile cognitive impairment, in particular, senile dementia.

However, a conventional cognitive training has been performed by classifying the user only based on a user brain state without considering characteristics of an individual user. Therefore, it has been ignored that even dementia users in the same state may have a question item activating the brain and its difficulty level that are different based on a user history.

Therefore, there is a need for a magnetic stimulation system which may accurately position the coil over the brain region to be stimulated to perform the transcranial magnetic stimulation. In addition, there is a need for personalized cognitive training which may adjust the item and level in consideration of user data so that the cognitive training performed together with the magnetic stimulation may practically and continuously activate a user's brain. Document US 2007/260107 A1 discloses systems for stereotactic transcranial magnetic stimulation of the brain to modulate neural activity at arbitrary deep and superficial brain locations. Document US 2010/087698 A1 discloses a method of using repetitive transcranial magnetic stimulation to treat a muscle group involved in a movement disorder.

### [Disclosure]

### [Technical ProblemSolution]

The present disclosure is devised to solve at least some of the problems of the prior art as described above, and an object of the present disclosure is to provide a magnetic stimulation system and method for detecting a motor threshold of a user by changing magnetic field output, thereby determining the magnetic field output suitable for each user, and a personalized cognitive training method and device for performing personalized cognitive training by using data for each user and a structured database.

### [Technical Solution]

The invention is defined by the appeneded claims. According to a first aspect of the present disclosure, a magnetic stimulation system comprising a magnetic field generation device,wherein the magnetic field generation device includes:a control unit determining a corresponding magnetic field output in consideration of user data including a motor threshold and a brain region to be stimulated; and a power module connected to a coil generating a magnetic field and supplying power for the coil to generate the determined magnetic field, and the control unit includes:a motor threshold measurement module measuring the motor threshold of a user by using a magnetic field change module; and the magnetic field change module controlling the magnetic field output by adjusting a magnetic field-related parameter, wherein the motor threshold measurement module measures the motor threshold of the user by stepwise adjusting a magnetic field intensity by using the magnetic field change module, and measures the motor threshold of the user by increasing a magnetic field frequency by a predetermined ratio and then stepwise adjusting the magnetic field intensity again when the magnetic field intensity reaches a maximum magnetic field output, the magnetic field change module determines the magnetic field output based on the measured motor threshold and a distance of the treatment position to the brain region to be stimulated, and a user brain state including at least one of user brain shape, brain size, and head shape, acquired using a received magnetic resonance imaging (MRI) image of the user.

According to a second aspect of the present disclosure, a magnetic stimulation method, performed by a magnetic field generation device including a control unit determining a corresponding magnetic field output in consideration of user data including a motor threshold and a brain region to be stimulated, and a power module connected to a coil generating a magnetic field and supplying power for the coil to generate the determined magnetic field output, may include: measuring a motor threshold of a user by adjusting the intensity or frequency of a magnetic field generated by a coil; positioning the coil to correspond to a head position corresponding to a position of the brain region to be stimulated,performing brain stimulation by driving the coil based on magnetic field data determined in consideration of the measured motor threshold of the user and a distance of a treatment position to the brain region to be stimulated, and a user brain state including at least one of user brain shape, brain size, and head shape, acquired using a magnetic resonance imaging (MRI) image of the user,wherein the measuring of the motor threshold of the user includes: a first level of performing magnetic field stimulation on a motor cortex region of the user by the coil to check whether a finger movement occurs or to check whether a change in user motility occurs using a motor threshold measurement sensor; a second level of increasing the magnetic field intensity by a predetermined ratio until a change in the finger movement or motility occurs when no finger movement or no change in the motility occurs; and a third level of increasing the magnetic field frequency when the magnetic field intensity reaches a maximum magnetic field output, and returning to the second level.

According to a third aspect of the present disclosure, a personalized cognitive training method, performed by a cognitive training device may include: presenting, by a question presenting unit, a predetermined type of question based on a brain region to be trained when user data is input to the device (here, the questions are classified based on their levels and types); determining, with a score determination unit, a cognitive-training result score based on user feedback on the presented question; anddetermining, with a question change unit, a question of a next round based on the cognitive-training result score of a current round, wherein in the determining of the question of the next round, a question type of the next round is changed based on the cognitive-training result score.

According to a fourth aspect of the present disclosure, a personalized cognitive training device performing a plurality of cognitive training may include: a content storage unit classifying and storing a content used for presenting a question for each level or item; a question presenting unit presenting the corresponding question for cognitive training in consideration of user data and a brain region to be activated through the cognitive training (here, the questions are classified based on their levels and types) ; a score determination unit determining a range of a score acquired from the questions presented by the question presenting unit or determining whether to maintain the score; and a question change unit changing a question level or a question pool based on a result of the score determination unit; wherein the question change unit includesa level adjustment module adjusting the question level to maintain a predetermined optimal score range, and a pool change module changing a question type of a next round based on the acquired cognitive-training result score.

According to a fifth aspect of the present disclosure, provided is a computer-readable recording medium recording a program for executing the magnetic stimulation method according to a second aspect or the personalized cognitive training method according to a third aspect on a computer.

### [Advantageous Effects]

The magnetic stimulation system according to the exemplary embodiments of the present disclosure may secure the higher treatment efficiency by adjusting the magnetic field output to the threshold value of the motor threshold that is acquired by always measuring a motor threshold of the user before performing the magnetic stimulation, and performing the magnetic stimulation to maximize the brain stimulation efficiency without the user movement based thereon.

In addition, the personalized cognitive training method and device according to the exemplary embodiments of the present disclosure, performed simultaneously or alternately with the above-mentioned magnetic stimulation, may also secure higher brain activation efficiency and user treatment efficiency by adjusting and presenting the questions by type, level, and item to stimulate the brain based on the relevance to the user data to thus perform the personalized cognitive training for each user.

### [Description of Drawings]

FIG. 1 is a schematic diagram illustrating a coil corresponding to a human brain to explain a magnetic stimulation method.
FIG. 2 is a schematic block diagram illustrating a magnetic field generation device according to an exemplary embodiment of the present disclosure.
FIG. 3 is a schematic block diagram illustrating a configuration of a brain navigation device according to an exemplary embodiment of the present disclosure.
FIG. 4 is a schematic block diagram illustrating a configuration of an entire magnetic stimulation system including the magnetic field generation device and the navigation device according to an exemplary embodiment of the present disclosure.
FIG. 5 is a flow chart of a motor threshold measurement module measuring a motor threshold according to an exemplary embodiment of the present disclosure.
FIG. 6 is a flow chart illustrating detection of a brain region to be stimulated according to an exemplary embodiment of the present disclosure.
FIG. 7 shows coordinates indicating data on a position of the brain region to be stimulated according to an exemplary embodiment of the present disclosure.
FIG. 8 is a schematic block diagram illustrating a configuration of an entire magnetic stimulation system including a magnetic field generation device using multi-coils and a navigation device according to another exemplary embodiment of the present disclosure.
FIG. 9 is a flow chart of the magnetic stimulation method according to an exemplary embodiment of the present disclosure.
FIG. 10 is a flowchart of a motor threshold detection method according to an exemplary embodiment of the present disclosure.
FIG. 11 is a schematic block diagram illustrating a configuration of a personalized cognitive training device according to an exemplary embodiment of the present disclosure.
FIG. 12 is a schematic block diagram illustrating a configuration of a content storage unit according to an exemplary embodiment of the present disclosure.
FIG. 13 shows a structure in which the content storage unit classifies and stores words according to an exemplary embodiment of the present disclosure.
FIG. 14 shows a structure in which a content storage unit classifies and stores words according to another exemplary embodiment of the present disclosure.
FIG. 15 is a flow chart of a personalized cognitive training method according to an exemplary embodiment of the present disclosure.
FIG. 16 is a flow chart illustrating a level adjustment of the personalized cognitive training method according to an exemplary embodiment of the present disclosure.
FIG. 17 is a flow chart illustrating the level adjustment and question pool adjustment of the personalized cognitive training method according to an exemplary embodiment of the present disclosure.
FIG. 18 is a flow chart illustrating a question pool adjustment of the personalized cognitive training method according to an exemplary embodiment of the present disclosure.
FIG. 19 is a flow chart illustrating the level adjustment and question pool adjustment of the personalized cognitive training method, performed in consideration of user data, according to an exemplary embodiment of the present disclosure.
FIG. 20 is a schematic block diagram illustrating the configuration and operation of a control unit according to another exemplary embodiment of the present disclosure.
FIG. 21 shows a method of a word analysis and classification module classifying words according to another exemplary embodiment of the present disclosure.
FIG. 22 is a flowchart of a word analysis and classification module classifying words according to another exemplary embodiment of the present disclosure.

### [Best Mode]

Hereinafter, the present disclosure is described in detail with reference to the accompanying drawings. However, the present disclosure is not limited to specific embodiments described below, and may be modified in various different forms. In the drawings, shapes and dimensions of elements may be exaggerated for clarity, and the same components are denoted by the same reference numerals.

Hereinafter, a user may refer to a person who receives magnetic stimulation, controls a magnetic stimulation system to receive the magnetic stimulation, receives personalized cognitive training, or controls a personalized cognitive training device to receive the personalized cognitive training. A patient with cognitive impairment, such as a patient with senile dementia or a patient with other types of cognitive impairment, may be only an example of the user, and the user is not limited to the patient. In addition, the user may include a user who receives the magnetic stimulation and the user who uses the device to receive the magnetic stimulation. Hereinafter, the user may mainly indicate the user who receives the magnetic stimulation.

In addition, the present disclosure may be used for the cognitive training performed for various reasons, and may be used for a purpose of treatment or prevention by helping a child's brain development or helping brain activation when brain development is degraded due to the cognitive impairment.

In particular, the present disclosure may be used to treat and prevent the senile cognitive impairment. Senile cognitive impairment may include memory impairment indicating memory loss, or a dementia disease such as Alzheimer's neurodegenerative disease, and the present disclosure may prevent lower brain activity through the cognitive training, and treat and prevent diseases such as the cognitive impairment.

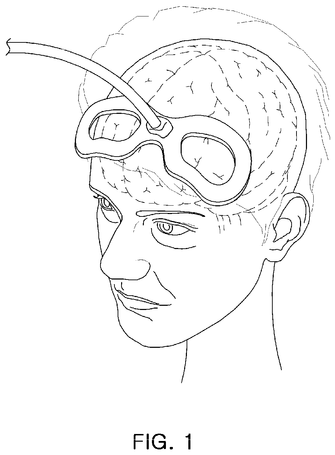

FIG. 1 is a schematic diagram illustrating a coil corresponding to a human brain to explain a magnetic stimulation method. As shown in FIG. 1, the magnetic stimulation method may include transcranial magnetic stimulation (TMS), transcranial direct current stimulation (TDCS), or the like. In particular, the TMS method is a non-surgical brain stimulation method in which nerve cells are activated by stimulating a specific region of the brain by using a magnetic field generated by a conductive electromagnetic coil. When a strong current is applied to the coil for the magnetic field to be generated, a strong magnetic field generated from the coil positioned to correspond to a user head may induce a biocurrent in the nerve cells of a user brain, and the induced current may stimulate the brain nerve cells.

Therefore, the magnetic stimulation method may provide a treatment that stimulates the brain as a psychiatric or neurological treatment, in which the nerve cells in the brain are not properly activated, such as depression or dementia treatment.

In particular, in the magnetic stimulation method, a figure-eight coil may be used rather than a circular coil to make a tip of the magnetic field sharp at a contact point between both the coils, thus making local stimulation possible through this configuration.

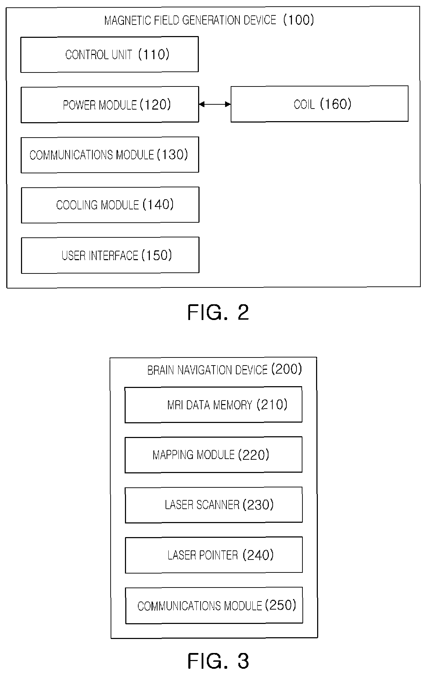

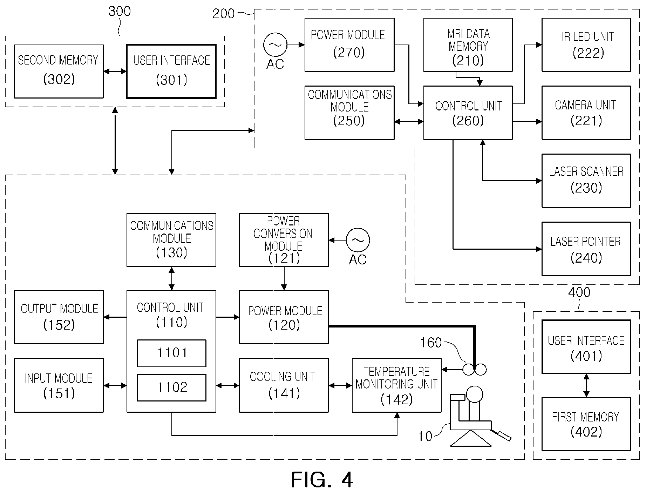

FIG. 2 is a schematic block diagram illustrating a magnetic field generation device according to an exemplary embodiment of the present disclosure; FIG. 3 is a schematic block diagram illustrating a configuration of a brain navigation device according to an exemplary embodiment of the present disclosure; and FIG. 4 is a schematic block diagram illustrating a configuration of the entire magnetic stimulation system including the magnetic field generation device and the navigation device according to an exemplary embodiment of the present disclosure.

Hereinafter, the description describes the entire magnetic stimulation system and each device in the magnetic stimulation system.

### Magnetic stimulation system

As shown in FIG. 4, the magnetic stimulation system may include a magnetic field generation device 100, a brain navigation device 200, an integrated operator 300 and a cognitive training device 400.

A coil 160 may be disposed on a side where the user brain is positioned based on a chair 10 on which the user sits, and the coil 160 and the magnetic field generation device 100 may be linked with each other.

The magnetic field generation device 100 according to an exemplary embodiment of the present disclosure may be a device determining magnetic field output and provides the same to the coil 160 for the magnetic field stimulation to be performed on the user brain.

The magnetic field generation device 100 according to an exemplary embodiment of the present disclosure may include a control unit 110 determining a corresponding magnetic field in consideration of user data including a motor threshold (MT) and a brain region to be stimulated, and a power module 120 connected to the control unit 110 and the coil 160 generating the magnetic field, and supplying power for the coil 160 to generate the determined magnetic field. The magnetic field generation device 100 may include a power conversion module 121 converting the magnetic field to alternating current (AC) power and supplying the same to the power module 120.

In addition, according to an exemplary embodiment of the present disclosure, the magnetic field generation device 100 may include: a user interface 150 including an output module 152 displaying an output magnetic field to the user who controls the magnetic field generation device 100, and an input module 151 allowing the user to manually adjust the magnetic field output; a communications module 130 communicating with the integrated operator 300 and the brain navigation device 200; and a cooling module 140 including a temperature monitoring unit 142 monitoring a temperature of the coil 160 and a cooling unit 141 cooling the magnetic field generation device 100 when the temperature exceeds a threshold value.

In addition, the brain navigation device 200 according to an exemplary embodiment of the present disclosure may be a device detecting a position of the brain region to be stimulated in linkage with the magnetic field generation device 100 for the magnetic field generation device 100 to be accurately positioned in the position of the user brain region to be stimulated, and providing data on the position of the brain region to the magnetic field generation device 100 to guide a position of the magnetic field generation device 100.

The brain navigation device 200 according to an exemplary embodiment of the present disclosure may include a magnetic resonance imaging (MRI) data memory 210 receiving and storing an MRI image of the user and the data on the position of the brain region to be stimulated, and mapping modules 221 and 222 capturing an actual user head image in real time by using the camera unit 221, and mapping the actual head image captured in real time with the MRI image of the user brain that displays the data on the position of the brain region to be stimulated that is stored in the MRI data memory 210.

In addition, the brain navigation device 200 may further include a laser scanner 230 scanning the user head to detect a head position corresponding to the position of the brain region to be stimulated, and a laser pointer 240 displaying the head position corresponding to the position of the brain region that is detected by the laser scanner 230.

Alternatively, the brain navigation device 200 may move a robot arm to the head position captured using the camera unit 221 to detect the head position corresponding to the position of the brain region to be stimulated.

That is, in an exemplary embodiment, the brain navigation device 200 may include the MRI data memory 210 and the mapping modules 221 and 222, further include the laser scanner 230 and the laser pointer 240 to easily detect the position of the brain region to be stimulated on the actual user head, or further include the robot arm receiving the position of the brain region to be stimulated.

The data on the position of the brain region to be stimulated may be position data manually detected by an expert such as a doctor from the MRI image of the user, or position data automatically detected using a trained artificial intelligence. The position data may be displayed as coordinates of X, Y, and Z axes.

The mapping module may include an infrared light emitting diode (IR LED) unit 222 emitting light to the user brain and the camera unit 221 receiving reflected light from light emitted by the IR LED unit 222 to mark the brain region to be stimulated. Here, the camera unit 221 may include a three dimensional (3D) camera. The brain navigation device 200 may guide the magnetic field generation device 100 to an accurate position to be stimulated using the mapping module.

In addition, the brain navigation device 200 may further include a communications module 250 performing communications to be linked with the magnetic field generation device 100, a control unit 260 controlling the above-described components, and a power module 270 supplying power to the control unit 260.

In addition, the cognitive training device 400 according to an exemplary embodiment of the present disclosure may stimulate the user brain by being driven simultaneously or alternately with the magnetic field generation device 100, and further activate a brain region stimulated by the magnetic field generation device 100 or complementarily activate a brain region less stimulated by the magnetic field generation device 100, thereby improving dementia treatment efficiency.

The cognitive training device 400 according to an exemplary embodiment of the present disclosure may include a first memory 402 storing user treatment data such as user personal data, a cognitive training question performed by the user, or a cognitive training score, and a user interface 401 presenting the cognitive training question and allowing the user to answer to the question as feedback.

The user interface 401 may include various user devices such as a desktop computer, a laptop computer, a personal digital assistant (PDA), and a tablet personal computer (PC), and may be a movable PC to improve user convenience.

The integrated operator 300 according to an exemplary embodiment of the present disclosure may be a device linked with the cognitive training device 400 or the magnetic field generation device 100, and controlling its driving time, time interval, frequency, intensity, or the like when simultaneously or alternately driving the cognitive training device 400 and the magnetic field generation device 100.

The integrated operator 300 may include a second memory 302 storing a user treatment history or personal data in preparation for loss of the user data in the first memory 402, and a user interface 301 through which the user capable of controlling the magnetic stimulation system, such as a doctor or a nurse, may manually control or check the driving time, the time interval, the frequency, the intensity, or the like.

The second memory 302 may be implemented as a cloud server type database (DB) as well as an internal storage device or an external storage device for the user treatment history or the input user data to be automatically linked to and stored in the cloud server. In particular, the second memory 302 may integrate and store the user data during remote medical treatment, thus facilitating data management and improving the user convenience.

The user interface 301 may include various user devices such as a desktop computer, a laptop computer, a personal digital assistant (PDA), and a tablet personal computer (PC), and may be a movable PC to improve user convenience.

### Magnetic field generation device 100

In order to use the magnetic stimulation method, the user is required to be still, that is, the user needs to make no movement, and simultaneously, the brain is required to be stimulated. Therefore, the magnetic field generation device 100 is required to maintain the magnetic field output close to the motor threshold (MT) to increase brain stimulation efficiency while adjusting the magnetic field output to be below the MT where the movement occurs in a user finger or the like.

A conventional magnetic stimulation method does not check the motor threshold separately, or checks the motor threshold by arbitrarily adjusting the intensity at a fixed magnetic field frequency. Therefore, the conventional magnetic stimulation method fails to provide personalized magnetic stimulation for each user because the user cannot proceed with further treatment when the user movement occurs at the arbitrary intensity.

In particular, the motor threshold may be changed based on the user's physical condition as well as the user's race, gender, or the like. Therefore, it is very necessary to accurately check the motor threshold right before the treatment for accurate and safe treatment.

To this end, as shown in FIGS. 2 and 4, the magnetic field generation device 100 according to an exemplary embodiment of the present disclosure may include the control unit 110 determining the corresponding magnetic field in consideration of the user data including the motor threshold (MT) and the brain region to be stimulated, and the power module 120 connected to the coil 160 generating the magnetic field, and supplying power for the coil 160 to generate the determined magnetic field.

The control unit 110 may include a magnetic field change module 1101 controlling the magnetic field output by adjusting a magnetic field-related parameter, and a motor threshold measurement module 1102 measuring the motor threshold (MT) of the user by using the magnetic field change module 1101.

Here, the magnetic field-related parameter may include at least one of its frequency, time, period, and intensity.

The motor threshold measurement module 1102 according to an exemplary embodiment of the present disclosure may measure the motor threshold of the user by stepwise adjusting the magnetic field intensity by using the magnetic field change module 1101. Alternatively, when the magnetic field intensity reaches the maximum magnetic field output, the motor threshold measurement module 1102 may measure the motor threshold of the user by increasing the magnetic field frequency by a predetermined ratio and then stepwise adjusting the magnetic field intensity again.

The motor threshold measurement module 1102 may check whether the coil 160 stimulates a motor cortex region in charge of a motor region of the brain, and then detect a user hand movement or a change in the pulse, muscle or nerve to determine whether the stimulation is being delivered to the brain.

In detail, the motor threshold of the user may be different for each person, and the motor threshold of the same user may be different for each day and for each condition. In particular, Asians may have much higher motor thresholds than Westerners. Accordingly, in a conventional magnetic stimulation device without a control method for accurate motor threshold measurement, the user cannot receive the magnetic stimulation treatment any longer when no finger movement occurs in the arbitrarily provided magnetic field output, or when a motor threshold measurement sensor does not recognize the change in the pulse, muscle or nerve.

On the other hand, the motor threshold measurement module 1102 according to an exemplary embodiment of the present disclosure may first set moderate magnetic field intensity or magnetic field frequency in a range of possible magnetic field intensities or magnetic field frequencies and then output the magnetic field. The motor threshold measurement module 1102 may set a reference point to measure the motor threshold of the user.

In an exemplary embodiment of the present disclosure, a motor threshold measurement sensor may include an electrocardiogram (ECG) sensor, an electromyography (EMG) sensor or the like.

Then, when no finger movement occurs in a first level, the ECG sensor fails to measure the change in the pulse, or the EMG sensor fails to measure a change in a current, the motor threshold measurement module 1102 may fix the magnetic field frequency, and increase and adjust the magnetic field intensity by a predetermined ratio R1 to thus increase the magnetic field intensity until the finger movement occurs, the ECG sensor measures the change in the pulse, or the EMG sensor measures the change in the current. Here, when detecting user motility, the motor threshold measurement module 1102 may determine that the intensity exceeds the motor threshold in which the motor cortex of the user brain shows reaction, and detect a threshold value immediately before reaching the motor threshold by reducing the frequency or intensity by a predetermined ratio R2 set smaller than the predetermined ratio R1. The threshold value may be a reference value of the magnetic field intensity to be used for the brain stimulation.

That is, there is a change in the motility when the change in the current occurs due to the change in the pulse or stimulation applied to the muscle or the nerve, and there is no change in the motility in the opposite case.

Meanwhile, even when the magnetic field intensity is increased to reach to the maximum magnetic field intensity, no finger movement may occur, the ECG sensor may measure no change in the pulse, or the EMG sensor may measure no change in the current. In this case, the motor threshold measurement module 1102 may increase the magnetic field output by increasing the magnetic field frequency. The motor threshold measurement module 1102 may increase the magnetic field frequency by a predetermined ratio R3, then return the magnetic field intensity to an initial intensity, and output the moderate magnetic field intensity. When detecting no motility, the motor threshold measurement module 1102 may increase and adjust the magnetic field intensity again by the predetermined ratio R1 to increase the intensity until the finger movement occurs, the ECG sensor measures the change in the pulse, or the EMG sensor measures the change in the current.

In this way, the motor threshold measurement module 1102 may allow every user to detect the motor threshold (MT) suitable for the user, set the threshold value immediately before reaching the motor threshold as the reference value of the magnetic field intensity that is to be used for the brain stimulation. Accordingly, the magnetic stimulation may be performed with the maximum magnetic field output to perform the brain stimulation because there is no user motility while the user is controlled not to move while receiving the brain stimulation, thereby maximizing the treatment efficiency while securing user safety.

Meanwhile, the sensor detecting the finger movement described above, the ECG sensor, or the EMG sensor is only an example, the scope of claims is not limited thereto, and any motor threshold measurement sensor detecting the user motility may be included in the claims of the present disclosure.

The magnetic field change module 1101 according to an exemplary embodiment of the present disclosure may determine the magnetic field output by using the received MRI image of the user, based on data on a treatment position displayed on the MRI image, a distance of the treatment position to the brain region to be stimulated, and a user brain state including at least one of user brain shape, brain size, and head shape.

The magnetic field generation device 100 may be linked with the brain navigation device 200 by communications performed through the communications module to receive the MRI image of the user that is stored in the brain navigation device 200, check the distance from a user scalp to the brain region to be stimulated and a user brain shape considering the deformation, such as how much the user brain is reduced, and adjust the magnetic field-related parameter to change the magnetic field output to the corresponding magnetic field output.

In particular, the magnetic field change module 1101 may be combined with an artificial intelligence module to learn the magnetic field-related parameter by considering a different brain shape for each person, a brain state based on a progress of a disease history, and a distance difference based on the brain region to be stimulated, and immediately output the corresponding magnetic field intensity reflecting the above considerations with no need to control and find the corresponding magnetic field one by one before performing the magnetic stimulation. In this way, it is possible to predict magnetic field data in advance to reduce time required for setting the magnetic field generation device 100 before the treatment while securing more treatment time, thereby improving the treatment efficiency.

In addition, the magnetic field change module 1101 according to an exemplary embodiment of the present disclosure may perform the magnetic field stimulation by driving the coil with the magnetic field intensity determined by calculating the motor threshold of the user that is measured at the predetermined ratio based on the brain region to be stimulated and the user brain state.

The reference value, which is the motor threshold value measured by the motor threshold measurement module 1102, may be the magnetic field output that maximizes the brain stimulation while preventing the human motility. However, there is a need to adjust the magnetic field output based on the brain region to be stimulated due to a difference in its position with that of the motor cortex of the brain that determines the motor threshold.

Therefore, the predetermined ratio may be set for each brain region based on the reference value by considering the position or distance of the brain region to be stimulated and the distance of the brain region to be stimulated based on the shape or size of the user brain, and each brain region may be stimulated by driving the coil with the magnetic field intensity acquired by multiplying the reference value by the ratio.

For example, the brain regions to be stimulated may be a total of 6 regions including Broca, Werniche, left and right dorsolateral prefrontal cortexes (DLPFC), and left and right parietal somatosensory association cortexes (PSAC).

Here, a ratio of the Broca region may be predetermined to 0.8 to 1.0 of the reference value. When the brain region to be stimulated is the Broca region, the magnetic field intensity applied thereto may be determined as a value acquired by multiplying the reference value by a ratio selected from the ratio of 0.8 to 1.0 of the reference value in consideration of the user brain state.

Meanwhile, the left and right pSACs are positioned at the rear side of the brain and are greatly reduced due to the diseases such as dementia. Therefore, a ratio of the left or right pSAC may be predetermined to 1.0 to 1.2 of the reference value, which is a higher ratio than the Broca region, and the magnetic field intensity applied thereto may be determined as a value acquired by multiplying the reference value by a ratio selected from the ratio of 1.0 to 1.2 of the reference value in consideration of the user brain state.

Meanwhile, a ratio of the left or right DLPFC may be predetermined to 0.9 to 1.1 of the reference value, and a ratio of the Wernicke region may be predetermined to 0.9 to 1.1 of the reference value. The predetermined ratio may be determined by considering the distance from the scalp to each brain region or the brain shape.

In particular, the user data including the user brain state to be considered may indicate the brain shape acquired by analyzing the MRI image of the user brain, the distance from the scalp to the brain region to be stimulated, and a user skull.

Therefore, the magnetic field change module 1101 may provide different magnetic stimulation by adjusting the magnetic field intensity, frequency, or the like based on the brain region to be stimulated and the user brain state.

Meanwhile, the motor threshold measurement module 1102 and the magnetic field change module 1101 are only classified based on their functions. The motor threshold measurement module 1102 and the magnetic field change module 1101 may be positioned together in one device, positioned on different devices and exist within one entire system, or positioned in different devices or systems to transmit and receive the data.

For example, there may be a separate device measuring the motor threshold and a separate device performing the magnetic field change to perform the magnetic field stimulation. In this case, the magnetic field change may be performed using the motor threshold measured by the separate device measuring the motor threshold, which may fall within the scope of the present disclosure.

In addition, for example, there may be a device measuring the motor threshold and a device performing the brain treatment through the magnetic field stimulation. In this case, the device performing the brain treatment may receive the motor threshold measured by the device measuring the motor threshold, or perform the brain treatment using the received motor threshold, which may also fall within the scope of the claims of the present disclosure.

Therefore, as shown in FIGS. 2 and 4, the magnetic field generation device 100 may include the control unit 110, the power module 120, and the coil 160, and may further include the communications module 130 performing the communications with the brain navigation device 200, the integrated operator 300, and the like.

The communications module 130 may include wired communications as well as wireless communications, and also use wireless fidelity (Wi-Fi) or the like.

In addition, device temperature management, coil temperature management in particular, may be very important to stably apply the magnetic stimulation to the user. Therefore, the magnetic field generation device 100 may further include the cooling module 140 including: the temperature monitoring unit 142 monitoring a change in the temperature of the coil 160 and the cooling unit 141 cooling the device when the temperature checked by the temperature monitoring unit 142 exceeds a threshold temperature.

In addition, the magnetic field generation device 100 may include the user interface 150 including the input module 151 and the output module 152 for the user who controls the magnetic field generation device 100 as the medical personnel to check or control the device 100 while the user is being treated.

Meanwhile, the magnetic field generation device 100 may request a manager's identity (ID) and password before performing the magnetic field stimulation for only a person permitted to treat the user to access the magnetic field generation device 100. Here, the manager indicates a person who controls the magnetic field generation device 100 for the user to use the magnetic field stimulation method, and the manager and the user may be the same person or different people.

Therefore, the manager granted only a magnetic field stimulation ONLY mode is allowed to only check data on the magnetic field stimulation performed on the user, and is not allowed to check other data such as cognitive training data.

### Brain navigation device 200

The brain navigation device 200 may analyze the MRI image of the user to detect the position of the brain region to be stimulated and provide the position to the magnetic field generation device.

As shown in FIGS. 3 and 4, the brain navigation device 200 according to an exemplary embodiment of the present disclosure may include the MRI data memory 210 receiving and storing the MRI image of the user and the data on the position of the brain region to be stimulated, and the mapping module 220 capturing the actual user head image in real time by using the 3D camera and mapping the MRI image of the user brain with the captured actual head image.

Here, the brain navigation device 200 according to an exemplary embodiment of the present disclosure may include the laser scanner 230 scanning the user head to detect the head position corresponding to the position of the brain region to be stimulated, and the laser pointer 240 displaying the head position corresponding to the position of the brain region that is detected by the laser scanner 230.

The position to be magnetically stimulated may be guided by the laser scanner 230 and the laser pointer 240.

Alternatively, in another exemplary embodiment of the present disclosure, the head position corresponding to the position of the brain region to be stimulated may be detected by a separate camera based the data mapped by the mapping module 220, and the robot arm connected to the magnetic field generation device 100 may be moved to the corresponding position.

Here, the robot arm may directly acquire the head position corresponding to the position of the brain region to be stimulated without a separate guide, and be moved to the corresponding position.

The mapping module 220 may include the IR LED unit 222 and the camera unit 221, and the above-described 3D camera may be included in the camera unit 221.

Separate medical personnel may manually find the data on the position of the brain region to be stimulated from the actual user head while viewing the mapped image by using the mapping module 220. Alternatively, the medical personnel may automatically acquire the data on the position of the brain region to be stimulated using the laser scanner 230 and the laser pointer 240.

In addition, the brain navigation device 200 according to an exemplary embodiment of the present disclosure may further include the communications module 250 performing the communications for the brain navigation device 200 to be linked with the magnetic field generation device.

According to an exemplary embodiment of the present disclosure, the position of the brain region may be followed by the laser scanner 230 linked with the 3D camera and detecting a person's movement, and the laser pointer 240 tracking a changed head position. Therefore, when the communications module 250 transmits a misalignment between a position of the coil and the head position tracked by the laser pointer 240, the control unit 110 of the magnetic field generation device 100 may stop driving the coil 160.

Alternatively, according to another exemplary embodiment of the present disclosure, the magnetic field generation device 100 may use the robot arm connected thereto. In this case, when the communications module 250 detects that the robot arm is out of the head position corresponding to the brain region to be stimulated, the control unit 110 of the magnetic field generation device 100 may stop the driving of the coil 160.

In detail, the position of the brain region to be stimulated is required to be detected in the actual brain of the user sitting on the chair 10 to receive the magnetic stimulation, and the coil 160 is then required to be positioned near a region of the head that corresponds to the corresponding position.

However, it may be very difficult to directly detect the position of the brain region to be stimulated from the actual brain, especially when the user is a dementia patient because the patient's brain is reduced and does not have a normal brain shape.

Therefore, the MRI image acquired by capturing the user brain may be stored in the MRI data memory 210 in advance. Here, the MRI image may be an image in which the treatment position is displayed by marking the user's nose tip, glabella, right and left ears, which are to be used as reference coordinates, and the position of the brain region to be stimulated is displayed based on these reference coordinates.

In addition, the brain navigation device 200 may further include an image quality improvement module receiving the MRI image of the user brain and improving an image quality before performing the markings on the MRI image. The pre-image processing may be performed because in case of the dementia patient with brain contraction, it is difficult to check the position of the brain region positioned at the rear side of the brain such as the PSAC even with the image even though it is less difficult to check the position of the brain region such as Broca. Through the pre-image processing, it is possible to detect more accurate position to thus increase the treatment efficiency.

The mapping module 220 may map the MRI image in which the treatment position is displayed with the actual user head currently captured by the 3D camera for the treatment position to be displayed on the actual head image, thereby guiding the magnetic field generation device 100 to the position of the brain region to be stimulated in the actual head.

The brain navigation device 200 may then use the laser scanner 230 to detect the actual head position corresponding to the brain region to be stimulated in the brain image of the real person sitting on the treatment chair 10 that is acquired through the 3D camera, allow the laser pointer 240 to adjust an internal mirror to irradiate a laser to the actual head position, or allow the robot arm to be automatically moved to the head position captured by the camera.

In this way, the user may determine the position of the coil 160 by matching an alignment reference point of the coil 160 with a point pointed by the laser pointer 240, or the robot arm may be automatically moved to the position of the coil 160. Accordingly, the user may avoid the hassle of searching for the brain stimulation region one by one while looking at a screen to thus have the improved convenience, and reduce time required to determine the position of the coil 160 to secure more treatment time, thus having the increased treatment efficiency.

That is, moving the position of the coil 160 may be manually performed by the user who controls the magnetic field generation device 100, or the position of the coil 160 may be moved using the robot arm.

When the robot arm is used, the data on the position of the brain region to be stimulated or coordinates of the actual head position corresponding to the data on the position of the brain region to be stimulated may be transmitted to the robot arm to move the robot arm in x, y, and z axes. Here, the robot arm may be directly driven based on the coordinates, and the laser pointer 240 separately displaying the position on the actual head may be omitted. However, it is possible to check the position movement of the robot arm by including the laser scanner 230 scanning the actual head to acquire data on the actual head position. It is also possible to omit the laser scanner 230 and directly transmit the coordinates to the robot arm for the robot arm to be moved to the position.

Therefore, the magnetic stimulation system according to another exemplary embodiment of the present disclosure may include the magnetic field generation device 100 including the coil 160 generating the magnetic field, the brain navigation device 200 detecting the position of the brain region to be stimulated by analyzing the MRI image of the user and guiding the position of the magnetic field generation device. Here, the brain navigation device 200 may include: the MRI data memory 210 receiving and storing the MRI image of the user and the data on the position of the brain region to be stimulated; the mapping module 220 capturing the actual user head image in real time by using the camera, and mapping the image of the real head that is captured in real time with the MRI image of the user brain that displays the position of the brain region to be stimulated that is stored in the MRI data memory; and the robot arm receiving the coordinates of the position of the brain region to be stimulated in the actual head that is acquired by the mapping module 220 and moving the coil 160 to correspond to the coordinates of the position.

That is, the brain navigation device 200 may check a user state through the camera when the user receiving the magnetic field stimulation breaks away from the magnetic field during the treatment, use the laser scanner 230 and the laser pointer 240 to easily return the user head to its original position corresponding to the coil 160 when the user moves out of the treatment position or the position of the head is misaligned, and check the user state through the camera and automatically move the robot arm to the identified position. The magnetic field generation device 100 may be linked with the brain navigation device 200 for the coil 160 to be turned off until the coil 160 returns to its original position.

That is, when positioned to correspond to the position of the brain region to be stimulated using the above components, the coil may be easily guided to the accurate position by the brain navigation device 200 linked with the magnetic stimulation system, and the magnetic stimulation system may be automatically stopped when the user makes the movement during the magnetic stimulation, thereby ensuring the user safety.

### Cognitive training device 400

According to another exemplary embodiment of the present disclosure, as shown in FIG. 4, the magnetic stimulation system may further include a cognitive training device 400 linked with the magnetic field generation device 100 to perform the cognitive training alternately or simultaneously with the magnetic field generation device 100 by presenting a predetermined question corresponding to the brain region to activate the brain region stimulated by the magnetic field generation device 100 after the brain is stimulated by the magnetic field generation device 100.

That is, the magnetic stimulation system may perform the magnetic field stimulation by using only the magnetic field generation device 100, but may increase the treatment efficiency by driving the cognitive training device 400 together.

For example, the brain regions performing cognitive functions may be a total of 6 regions including Broca, Werniche, left and right dorsolateral prefrontal cortexes (DLPFC), and left and right parietal somatosensory association cortexes (PSAC). This region is the same as the brain region where the brain stimulation is performed, and a cognitive training region and the brain region where the brain stimulation is performed may be different from each other.

The Broca region is in charge of the syntax, grammar, and expression of words, and the Werniche region is in charge of the meaning and classification of the words. In addition, the left and right DLPFs are in charge of action names, object names, or spatial memory, and the left and right PSACs are in charge of spatial or directional tasks. Therefore, a different type of cognitive training is required to be performed for each region in order to activate the region. The Broca region may perform selecting a similar sentence or selecting a correct or incorrect sentence, and the Werniche region may be activated when the user finds a correct word or a word matching a photo.

In addition, the left DLPFC may be activated when the user memorizes a word, finds a correct color of a figure, or finds a correct position of a figure, the right DLPFC may be activated when the user memorizes a word, finds a correct action state, or finds a correct object name, the left PSAC may be activated when the user finds a specific letter, and the right PSAC may be activated when the user finds colors and figures.

For example, finding a specific letter may be performed by illustrating an image in which a specific letter 'ga' is mixed with gya, geo, gyeo, or the like that is similar to 'ga,' and allowing the user to answer whether 'ga' is in the image. Here, the specific letter may not be in the image.

For another example, finding specified color and figure is a question form of finding corresponding color and figure in an image in response to the question 'Is there a vertical blue figure?'

Therefore, each brain region may be activated through the cognitive training in which the user solves the corresponding type of question based on the corresponding brain region, and one type of question may be presented to activate one brain region.

Alternatively, the magnetic field generation device 100 including multi-coils 160a and 160b described below may simultaneously perform the magnetic field stimulation on the plurality of brain regions. In this case, the cognitive training device 400 may also activate the plurality of brain regions by allowing the user to solve a cognitive training question of a mixed question type, which may simultaneously stimulate the plurality of corresponding brain regions.

That is, the magnetic field generation device 100 of the present disclosure may provide current stimulation to the nerve cells by outputting the magnetic field to the human brain region, and the cognitive training device 400 may activate the brain by allowing the user to solve the question type of the corresponding brain region to maximize the brain stimulation efficiency while the magnetic field generation device 100 performs the physical stimulation.

In addition, according to an exemplary embodiment of the present disclosure, the cognitive training may be performed separately at regular intervals, for example, every day, by using the personalized cognitive training device 400.

It is possible to simultaneously, alternately, or separately perform the magnetic stimulation by the magnetic field generation device 100 and the cognitive training by the personalized cognitive training device 400. However, it is preferable to perform the stimulation and the training simultaneously or alternately for the user convenience and time and cost saving.

The above-described cognitive training may be performed using the cognitive training device 400, and may be simultaneously or alternately performed periodically with the magnetic field generation device 100, thereby maximizing the brain stimulation efficiency. This training may treat or prevent the senile cognitive impairment, for example, the senile dementia, or may be applied to every user with the cognitive impairment, such as a child as well as an elderly man.

Meanwhile, the cognitive training device 400 may include a user DB storing the user data and a cognitive training DB storing the cognitive training data, and may access the DB through the cloud server. The cognitive training device 400 may perform the remote medical treatment through the cloud server to perform treatment at home. Coming to the hospital at regular intervals, for example every day, may cause user inconvenience. Accordingly, in this way, the treatment efficiency may be increased by increasing the user's treatment convenience and accessibility.

The first memory 402 may be implemented as a cloud server type database (DB) as well as an internal storage device or an external storage device for the user treatment history or the input user data to be automatically linked to and stored in the cloud server. In this case, the user data may be integrated and stored especially during the remote medical treatment, thus facilitating the data management and improving the user convenience.

That is, when the treatment data of the user who performed the cognitive training at home through the remote medical treatment is stored in first memory 402, the medical personnel may check contents of the first memory 402 directly through the cloud server, and also connect the first memory 402 to another server to automatically update the treatment data in the cloud server.

The description describes below a detailed configuration of the personalized cognitive training device 400 according to an exemplary embodiment of the present disclosure.

Therefore, the magnetic stimulation system according to an exemplary embodiment of the present disclosure may include the magnetic field generation device 100 and the brain navigation device 200, and may further include the cognitive training device 400.

### Integrated operator 300

As described above, the magnetic stimulation system of the present disclosure may further include the cognitive training device 400.

Here, as shown in FIG. 4, the magnetic stimulation system according to another exemplary embodiment of present disclosure may further include the integrated operator 300connected to the magnetic field generation device 100 and the cognitive training device 400 through the communications and simultaneously or alternately driving the magnetic field generation device 100 and the cognitive training device 400.

That is, the magnetic stimulation system may perform the magnetic field stimulation by using only the magnetic field generation device 100, but may increase the treatment efficiency by driving the cognitive training device 400 together, which may be controlled by the integrated operator 300.

The integrated operator 300 according to another exemplary embodiment of the present disclosure may perform the communications with the magnetic field generation device 100 or the cognitive training device 400, and the communications may be performed in a wired manner as well as a wireless manner such as Wi-Fi. The integrated operator 300 may determine related parameters such as a time interval between the cognitive training and the magnetic field stimulation, duration of the cognitive training, duration of the magnetic field stimulation, or the magnetic field stimulation intensity or frequency.

In detail, the integrated operator 300 may control the cognitive training performed by the cognitive training device 400 and the magnetic stimulation performed by the magnetic field generation device 100 to be performed simultaneously or alternately with each other.

In an exemplary embodiment, the magnetic stimulation may be performed by the magnetic field generation device 100, the cognitive training may then be performed by the cognitive training device 400 before an effect of the magnetic stimulation drops to a certain value or less, the cognitive training may then be performed by the cognitive training device 400, and the magnetic stimulation may be repeated again to thus periodically perform the plurality of cognitive trainings and the plurality of magnetic stimulations.

For example, the magnetic field generation device 100 may be a transcranial magnetic stimulation (TMS) device and stimulate the Broca region. In this case, the TMS device may stimulate 20 pulses for 2 seconds, and after the magnetic stimulation is finished, the cognitive training may perform a total of 20 sets with one set of solving 2 questions of 'finding similar sentences' for 28 seconds.

Here, the integrated operator 300 may set the cognitive training and the magnetic stimulation to be performed for 30 seconds for each set, set the TMS stimulation to be performed by 20 pulses for 2 seconds at 30 second intervals, and set the cognitive training for the user to solve 2 questions for 28 seconds, that is, 1 question for 14 seconds and control the question set to be repeated 20 times.

The above parameters may be changed based on the brain region to be stimulated, a type of the cognitive training question, the user data, or the like. For example, even though only the question type is changed to 'selecting correct or incorrect sentence' under the same condition as the above example, the integrated operator 300 may change the time interval of the TMS stimulation to 33 second intervals, and control the cognitive training setting to be changed to 'solving 2 questions for 31 seconds.'

For another example, the magnetic field generation device 100 may be the TMS device and stimulate the Werniche region. In this case, the TMS device may stimulate 20 pulses for 2 seconds, and after the magnetic stimulation is finished, the cognitive training may perform a total of 20 sets with one set of solving 4 questions of 'finding correct sentences' for 34 seconds.

Here, the integrated operator 300 may set the cognitive training and the magnetic stimulation to be performed for 36 seconds for each set, set the TMS stimulation to be performed by 20 pulses for 2 seconds at 36 second intervals, and set the cognitive training for the user to solve 4 questions for 36 seconds, that is,1 question for 8.5 seconds and control the question set to be repeated 20 times.

In addition, when the user is a patient with cognitive impairment, for example, a patient with senile dementia, the integrated operator 300 may increase the number of magnetic fields or the number of cognitive training in consideration of this situation.

Therefore, the integrated operator 300 may consider the brain region to be stimulated, the type of cognitive training question for stimulating the brain region, the treatment history that the user received so far, the personal data, the type of magnetic stimulation, or the like. Based on these considerations, the integrated operator 300 may adjust a total number of sets performed by the magnetic field generation device 100, the magnetic stimulation time, frequency, and rest time after the stimulation in one set, and adjust the number of cognitive training question items, solution time for each question, a total number of performance sets performed by the cognitive training device 400.

Meanwhile, the second memory 302 may be implemented as the cloud server type database (DB) as well as the internal storage device or the external storage device for the user treatment history or the input user data to be automatically linked to and stored in the cloud server. The integrated operator 300 may remotely adjust cognitive training protocols and magnetic stimulation protocols based on the above-described training or stimulation result.

Therefore, the magnetic stimulation system according to an exemplary embodiment of the present disclosure may include the magnetic field generation device 100 and the brain navigation device 200, and further include the cognitive training device 400 and the integrated operator 300. The integrated operator 300 may control the cognitive training to be performed between the previous magnetic field stimulation and the next magnetic field stimulation, and may also control the cognitive training to be performed simultaneously with the stimulation.

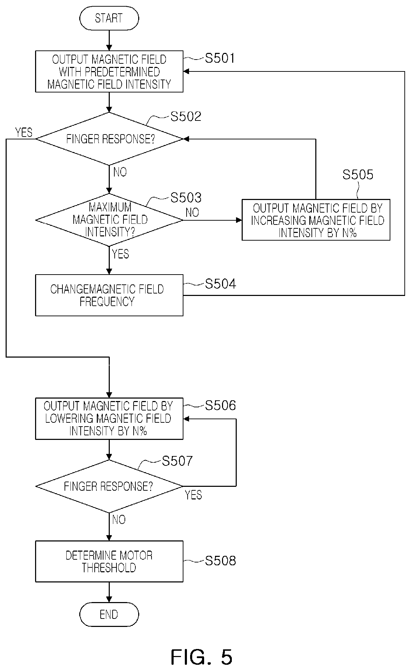

Meanwhile, FIG. 5 is a flow chart of the motor threshold measurement module 1102 measuring the motor threshold according to an exemplary embodiment of the present disclosure.

As shown in FIG. 5, in order to measure the motor threshold (MT), the magnetic field with a predetermined magnetic field intensity may be output to the coil 160 by using the magnetic field change module 1101 (S501).

The predetermined magnetic field intensity may indicate, for example, a mid-range magnetic field intensity at a current magnetic field frequency to set the reference point.

When the magnetic field is output with the predetermined magnetic field intensity, a finger response may not occur in which a user finger flinches or a user wrist bounces (No in S502). In this case, it may be considered that the magnetic field intensity has yet to exceed the motor threshold and the magnetic field intensity may be adjusted greater than before.

In another exemplary embodiment, it is possible to check whether the motor cortex of the brain is being stimulated by installing the ECG sensor to detect the change in the pulse or installing the EMG sensor to detect the change in the current, rather than detecting the finger movement occurring due to the magnetic field stimulation.

This configuration is an example, and a step S502 may be performed using another motor threshold measurement sensor capable of measuring the user motility.

Here, when magnetic field intensity is not the maximum magnetic field intensity which may be output (No in S503), the magnetic field intensity may be output by being stepwise increased by the predetermined ratio R1 (S505), and it may be continuously checked whether the finger response occurs (return to S502).

For example, when the predetermined ratio R1 is set to n% and n is 5%, the magnetic field intensity may be increased by 5% from the initially-predetermined magnetic field intensity and adjusted high until the intensity exceeds the motor threshold to cause the finger response.

Then, when there is no finger response yet, but the currently-output magnetic field has the maximum magnetic field intensity that may be output (Yes in S503), the magnetic field frequency may be changed to be high (S504).

The conventional system may use one fixed magnetic field frequency. Accordingly, when the motor threshold is an amount of the stimulation generated by the maximum magnetic field intensity or more, the user cannot receive the brain stimulation treatment any longer and may thus be excluded from the treatment.

On the other hand, according to an exemplary embodiment of the present disclosure, it is possible to increase the magnetic field frequency by changing the magnetic field mode (S504), and output the magnetic field with the predetermined magnetic field intensity again in a state where the magnetic field frequency is changed (return to S501).

Therefore, it may be considered that the intensity exceeds the motor threshold when the finger response occurs at a specific magnetic field frequency or intensity while repeating the steps S501 to S505 again (Yes in S502), and the motor threshold measurement module 1102 may lower the magnetic field frequency or intensity at the predetermined ratio R2 by using the magnetic field change module 1101 and output the same (S506).

In detail, when detecting the motility in which the finger response occurs, it may be determined that the frequency or the intensity exceeds the motor threshold to which the motor cortex of the user brain responds, and the frequency or the intensity may be reduced by the predetermined ratio R2 set smaller than the predetermined ratio R1 to thus detect the threshold value immediately before reaching the motor threshold. The reason is that although the brain stimulation is required to stimulate only the brain region to be stimulated, other regions may be stimulated when the user moves, and it is thus required to output the stimulation with the intensity to an extent that the brain is activated through the brain stimulation, with no user motility.

For example, when R1 is set to 5%, it is necessary to adjust the magnetic field intensity in more detail to detect the threshold value of the motor threshold. Therefore, it is possible to lower the magnetic field intensity by setting R2 = 1% (m = 1), and output the same.

Therefore, when the finger response occurs (Yes in S507), the magnetic field intensity may be continuously lower by m%, and when the finger response occurs no longer (No in S507), the magnetic field intensity here may be determined as the motor threshold (S508), and the motor threshold may be taken as the reference value of magnetic field intensity during the brain stimulation.

In this way, it is possible to perform a motor threshold detection method personalized for every user, and quickly and easily detects the motor threshold changed based on the user's race, gender, or daily condition, thereby ensuring the user safety and simultaneously maximizing the treatment efficiency.

In addition, although the above-described example is limited to the finger response, which is only an example, the present disclosure may also include an example in which the motor threshold response is checked using the motor threshold measurement sensor, and the scope of the claims is not limited thereto.

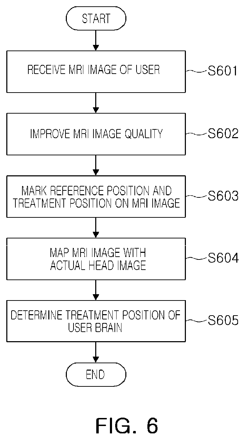

FIG. 6 is a flow chart illustrating detection of the brain region to be stimulated according to an exemplary embodiment of the present disclosure.

As shown in FIG. 6, the position of the brain region to receive the magnetic field stimulation may be detected using the brain navigation device 200 after a user-personalized motor threshold is detected as described with reference to FIG. 5.

The brain navigation device 200 may receive the MRI image of the user (S601). The received MRI image of the user may be stored in the MRI data memory 210.

Next, the MRI image quality may be improved (S602). The reason is that the MRI image of the user may have the lower image quality and it may thus then be difficult to map the MRI image with the actual brain image. The brain navigation device 200 may further include the image quality improvement module to improve MRI image quality by marking the reference coordinates and the brain region coordinates to be stimulated in advance. Alternatively, after improving the image quality, the brain navigation device 200 may mark the reference coordinates and coordinates of the treatment position (S603) and store the same in the MRI data memory 210.

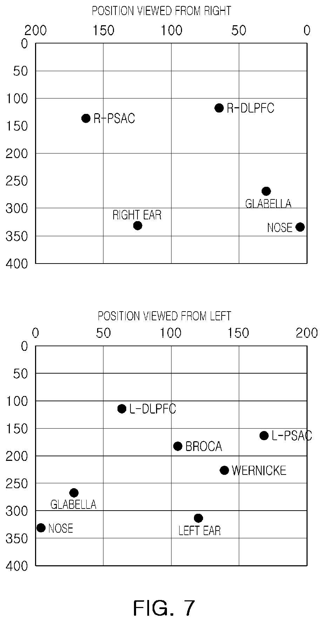

Here, FIG. 7 shows the reference coordinates and the coordinates of the brain region to be stimulated. The nose tip, glabella, right ear, and left ear of the user may be marked as the reference coordinates.The nose tip, the glabella, and the right ear may be the reference coordinates for the brain region positioned on the right side, and the nose tip, the glabella, and the left ear may be the reference coordinates for the brain region positioned on the left side.

The coordinates of the R-PSAC and R-DLPFC positions may be acquired based on the reference coordinates when viewed from the right side, and the coordinates of Broca, Werniche, L-PSAC, and L-DLPFC positions may be acquired based thereon when viewed from the left side.

The position data may be acquired based on the reference coordinates directly marked by the medical personnel capable of reading the MRI image, or acquired through the artificial intelligence module performing the learning and outputting the position data.

Therefore, when a clear MRI image is acquired, the MRI image with the actual head image may be mapped by the mapping module 220 (S604) to thus match the coordinates of the treatment position that are marked on the MRI image with the corresponding coordinates of the actual head image.

As the corresponding coordinates of the actual head image are detected, the treatment position of the user brain where the coil 160 is to be positioned on the actual user head may be determined by using the laser scanner 230 and the laser pointer 240 or by transmitting the detected coordinates to the robot arm (S605). That is, the determined coordinates may be pointed using the laser pointer 240 or transmitted to the robot arm for the magnetic field generation device 100 connected to the robot arm to be automatically moved.

That is, the position of the coil 160 that corresponds to the motor threshold for each user and the brain region to be treated may be detected through the above flow, andeach brain region may receive the magnetic field stimulation with the magnetic field intensity adjusted based on the motor threshold by the coil 160.

### Magnetic field generation device 100 including multi-coils 160a and 160b

Meanwhile, the magnetic field generation device 100 as described above may include one coil 160, and may thus stimulate one region in turn when stimulating the plurality of brain regions.

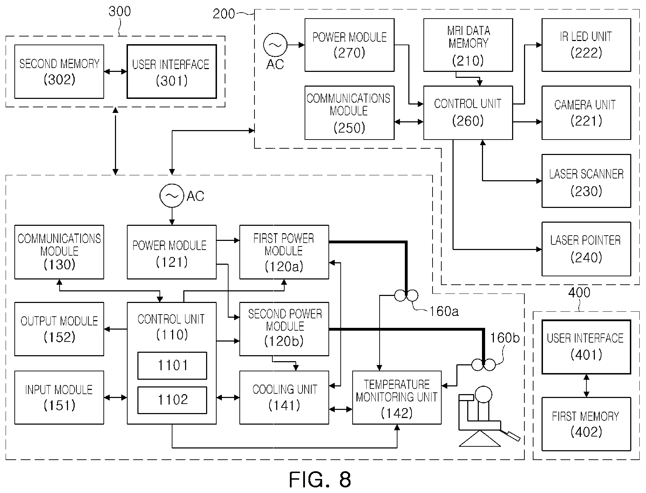

On the other hand, as shown in FIG. 8, the magnetic field generation device 100 including multi-coils 160a and 160b may simultaneously perform the brain stimulations by using the multi-coils 160a and 160b when stimulating the plurality of brain regions. Here, it is preferable that one multi-coil 160a may stimulate at least one of the right brain regions, and the other multi-coil 160b may stimulate at least one of the left brain regions.

That is, the multi-coils 160a and 160b may respectively be positioned to correspond to the brain region positioned on the right side of the user and the brain region positioned on the left side of the user, and perform the magnetic field stimulations on the brain region positioned on the right side and the brain region positioned on the left side simultaneously or with a time interval. The multi-coils 160a and 160b may perform the stimulations together without a time difference or separately with the time difference.

Here, the multi-coils 160a and 160b may simultaneously stimulate one brain region positioned on the right side and one brain region positioned on the left side, simultaneously stimulate two or more brain regions positioned on the right side, or simultaneously stimulate two or more brain regions positioned on the right side and one brain region positioned on the left side.

That is, the multi-coils 160a and 160b may perform the magnetic field stimulations on at least one brain region positioned on the right side or at least one brain region positioned on the left side simultaneously or with the time interval.

In addition, according to an exemplary embodiment of the present disclosure, when the multi-coils 160a and 160b perform the magnetic field stimulations simultaneously or with the time interval, the magnetic field stimulations may be performed by controlling the respective frequencies, periods, times, and intensities of the magnetic fields to be the same or different from each other based on the brain regions to be stimulated where the multi-coils 160a and 160b are respectively positioned.

For example, when the contracted brain region is focused on the Broca region, it is possible to perform the stimulation and the cognitive training on another brain region through a different channel while continuously performing the treatment on the Broca region. It is thus possible to perform the personalized treatment for each person by using the multi-coils 160a and 160b.

In addition, when simultaneously stimulating the left and right PSAC regions, the magnetic field stimulation may be performed by controlling the intensities, frequencies, and stimulation periods of the output magnetic fields to be the same as each other.However, when simultaneously stimulating the right PSAC region and the Broca region, the magnetic field stimulation may be performed by controlling the intensities, frequencies, and stimulation periods of the output magnetic field to be different from each other. In this way, even though the stimulations are simultaneously performed using the multi-coils 160a and 160b, it is possible to perform the personalized treatment for each brain region to be stimulated by performing the magnetic field stimulations by adjusting the intensities or the like to be different from each other for each position.

Therefore, according to an exemplary embodiment of the present disclosure, the magnetic field generation device 100 may include the control unit 110 determining the corresponding magnetic field in consideration of the user data including the motor threshold (MT) and the brain region to be stimulated, and power modules 120a and 120b respectively connected to the multi-coils 160a and 160b simultaneously stimulating the plurality of brain regions and supplying power to each of the multi-coils 160a and 160b. Each brain region may require a different intensity or protocol of the magnetic field, and the power modules 120a and 120b corresponding to the multi-coils 160a and 160b may be disposed in the power conversion module 121 to output optimal magnetic fields corresponding to the respective brain regions.

In addition, the control unit 110 may include the magnetic field change module 1101 controlling the respective magnetic field output by adjusting at least one of the frequencies, periods, times, and intensities of the respective magnetic fields generated by the multi-coils 160a and 160b, and the motor threshold measurement module 1102 measuring the motor threshold (MT) of the user by using the magnetic field change module 1101.

The components of another magnetic field generation device 100, such as the brain navigation device 200, the cognitive training device 400, and the integrated operator 300, are the same as those described above. However, as the plurality of brain regions are stimulated, during the cognitive training, the cognitive training device 400 may simultaneously perform the training on the plurality of brain regions to be simultaneously stimulated by the magnetic field generation device 100. In this case, the question which is the mixture of the pre-determined questions may be presented based on the brain region. Accordingly, the integrated operator 300 may also perform the control considering the magnetic field stimulation and the cognitive training on the plurality of brain regions.

Other descriptions are the same as those described above, and the same descriptions are thus omitted to avoid redundancy.

In this way, it is possible to simultaneously perform the brain stimulations on the plurality of regions, and perform more brain stimulations for the same time, thereby saving time and cost and increasing the treatment efficiency.

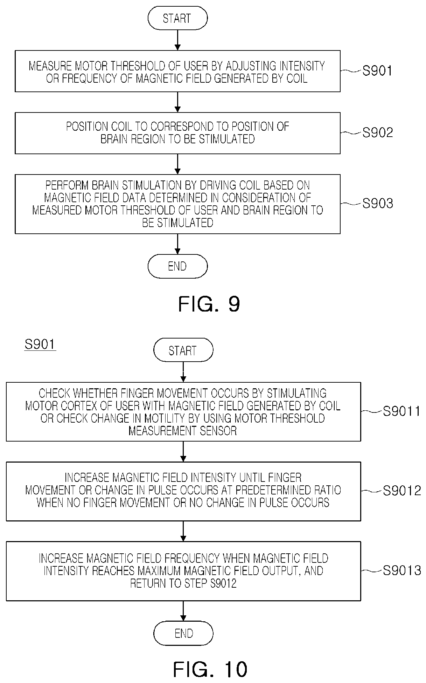

Meanwhile, FIG. 9 is a flow chart of the magnetic stimulation method according to an exemplary embodiment of the present disclosure; and FIG. 10 is a flowchart of the motor threshold detection method according to an exemplary embodiment of the present disclosure. The magnetic stimulation method below may be performed by the magnetic generating device 100 described above.

Therefore, as shown in FIG. 9,according to an exemplary embodiment of the present disclosure, the magnetic stimulation method may include: measuring a motor threshold of a user by adjusting the intensity or frequency of a magnetic field generated by a coil (S901); positioning the coil to correspond to a position of a brain region to be stimulated (S902); and performing brain stimulation by driving the coil based on magnetic field data determined in consideration of the measured motor threshold of the user and the brain region to be stimulated (S903).

In addition, as shown in FIG. 10, the measuring of the motor threshold of the user (S901) may include: checking whether a finger movement occurs by stimulating a motor cortex of the user with the magnetic field generated by the coil or checking user motility by using an electrocardiogram (ECG) sensor or an electromyography (EMG) sensor (S9011); increasing the magnetic field intensity until the finger movement or a change in a pulse occurs at a predetermined ratio when no finger movement or no change in the motility occurs (S9012); and increasing the magnetic field frequency when the magnetic field intensity reaches the maximum magnetic field output, and returning to the step S9012 (S9013).

In addition, according to an exemplary embodiment of the present disclosure, in the step S9012, when the finger movement or the change in the motility occurs, the magnetic field intensity may be lower to a threshold value where no finger response occurs or the motility returns to its previous state, and the threshold value may be determined as the motor threshold (MT).

In addition, specifically in the positioning of the coil to correspond to the position of the brain region to be stimulated (S902), data on the position of the brain region to be stimulated may be acquired by mapping, by a brain navigation device 200, a magnetic resonance imaging (MRI) image of the user that displays the position of the brain region to be stimulated with an actual brain image captured by a three dimensional (3D) camera, then a distance to the position of the brain region to be stimulated may be detected by a laser scanner 230, and a position of the brain region that is detected by a laser pointer 240 may be displayed.

Alternatively, according to another exemplary embodiment of the present disclosure, the data on the position of the brain region to be stimulated may be acquired, and then transferred to a robot arm, and the robot arm may be driven to be moved to the corresponding position captured by the 3D camera.

Therefore, according to an exemplary embodiment of the present disclosure, the positioning of the coil to correspond to a head position corresponding to the position of the brain region to be stimulated (S902) may include: mapping, by the brain navigation device 200, the MRI image of the user that displays the position of the brain region to be stimulated with an actual head image captured by the camera unit 220; detecting the head position corresponding to the position of the brain region to be stimulated displayed in the mapped image from the head image captured in real time by the laser scanner 230; and displaying the detected head position by the laser pointer 240 or transmitting the detected head position to the robot arm.

In addition, according to another exemplary embodiment of the present disclosure, the performing of the brain stimulation by driving the coil (S903) may further include a step of controlling, by a magnetic field change module 1101, at least one of the respective frequencies, periods, times, and intensities of the magnetic fields generated by multi-coils 160a and 160b when the plurality of brain regions are stimulated simultaneously by driving the multi-coils 160a and 160b.

Here, when magnetic field stimulation is performed by the multi-coils 160a and 160b simultaneously or with a time interval, the magnetic field stimulations may be performed by controlling the respective frequencies, periods, times, and intensities of the magnetic fields generated by the multi-coils 160a and 160b to be the same or different from each other by the magnetic field change module 1101.

Meanwhile, according to an exemplary embodiment of the present disclosure, the magnetic stimulation method may further include a step of performing cognitive training, by a separate cognitive training device 400, by presenting a predetermined question corresponding to the brain region for the brain region stimulated by the magnetic field generation device 100 to be activated.

When outputting the magnetic field, the above-mentioned magnetic field change module 1101 may adjust the frequency in a range of 1 to 200 Hz, adjust the intensity in a range of 1 to 10 tesla, and adjust the time in units of 1 to 200 ms. The above-described range is within an output range prescribed in safety regulations of the U.S. food and drug administration (FDA), and the magnetic field output may be changed within the above range to secure user safety while maintaining maximum output efficiency to increase treatment efficiency.

Therefore, in the performing of the brain stimulation by driving the coil (S903), the output magnetic field data may be adjusted within the above-described range.

In addition, the magnetic field generation device 100 according to an exemplary embodiment of the present disclosure may be a transcranial magnetic stimulation (TMS) device. In this case, the TMS device may be a repetitive transcranial magnetic stimulation (rTMS) device performing the repetitive magnetic stimulation, and the magnetic field change module 1101 may output the stimulation as theta-burst stimulation (TBS) such as continuous TBS (cTBS) or intermittent TBS (iTBS). The theta burst stimulation may be performed in a short period of time with ultra-high frequency stimulation, and the stimulation may be performed at a frequency of 50 Hz in the above-described range and at a time interval of 200 ms.

The magnetic field change module 1101 may adjust a stimulation frequency of the rTMS to maintain optimal brain-region stimulation efficiency.

Therefore, in the present disclosure, the brain may be stimulated with 1500 pulses for each day by using the above-described magnetic stimulation method, and three brain regions may be simultaneously stimulated when using the multi-coil 160. The number of pulses to perform the brain stimulation follows a TMS clinical trial and safety guidance.

Meanwhile, according to another exemplary embodiment of the present disclosure, the magnetic stimulation device may include a processor configured to perform the steps of the magnetic stimulation method described above; and a memory storing brain stimulation data in a cloud server, the brain stimulation data being acquired driving the coil based on the motor threshold of the user or the determined magnetic field data.

The magnetic stimulation device may perform the magnetic field stimulation by generating the magnetic field, and the memory may store both user data including data such as the user's gender, race, age, or the like, and data on the magnetic field stimulation performed on the user. The data may be later used to control the magnetic field stimulation to be performed on the corresponding user or used as reference data for another user having the same user data. Here, the memory is the cloud server and may be utilized for remote medical treatment.

In addition, the magnetic stimulation device may further include a transceiver performing remote communications to execute the above-described processor. When the processor executes the steps of the magnetic stimulation method, the robot arm may be driven correspondingly for the magnetic stimulation method to be executed, and the processor may perform the remote control through the transceiver for the robot arm to be remotely driven.

A remote medical treatment system according to another exemplary embodiment of the present disclosure may include: the magnetic stimulation system including at least one of the magnetic field generation device 100 performing the magnetic field stimulation, the brain navigation device 200 guiding the position of the magnetic field generation device 100, and a cognitive training device 400 performing cognitive training simultaneously or alternately with the magnetic field generation device 100; the cloud server storing a user usage result of the magnetic stimulation system; and a manager terminal connected to the cloud server through a security procedure to check the user usage result, and remotely controlling the magnetic field output or cognitive training of the magnetic stimulation system.

The magnetic stimulation system may be the magnetic stimulation system according to the above-described exemplary embodiment, the manager terminal may be a manager terminal having an identity (ID) and a password, authorized to access the cloud server, and the manager terminal may be a user interface such as a computer installed in a hospital.

According to another exemplary embodiment of the present disclosure, the manager terminal may control at least one of the frequency, period, and time of the magnetic field generated by the magnetic field generation device and the period and time of a question pool of the cognitive training performed by the above cognitive training device in consideration of at least one of the brain region to be stimulated by the magnetic stimulation system, the user data, and a question type of the cognitive training performed by the cognitive training device, and update a control content to the cloud server in real time. In this way, it is possible to integrate and manage a user treatment history, thereby facilitating data management and establishing a database (DB) .

Detailed contents updated in the cloud server are described below.

Meanwhile, when stimulating the user brain by the magnetic field generation device 100 of the magnetic stimulation system, the user brain may be provided with the brain stimulation by the training performed by the cognitive training device in addition to the direct stimulation by the magnetic field. It is possible to maximize brain stimulation efficiency by performing the cognitive training in which the stimulated brain is trained before brain activity generated by the stimulation is reduced after the brain is stimulated by the magnetic field generation device 100.

The personalized cognitive training device 400 according to an exemplary embodiment of the present disclosure may perform the cognitive training immediately after the brain stimulation is performed by the magnetic field generation device 100, before the brain activity generated by the brain stimulation is reduced to a certain value or less, or simultaneously with the brain stimulation performed by the magnetic field generation device 100.

Alternatively, the cognitive training by the personalized cognitive training device 400 may be performed several times at a certain period alternately with the stimulation performed several times at a certain period by the magnetic field generation device 100.

That is, the personalized cognitive training device 400 may be further used when using the magnetic field generation device 100. In this way, it is possible to increase user treatment efficiency by supplementing and maximizing the user brain stimulation efficiency, and improve user convenience by reducing the number of user visits to a specific location to receive the brain stimulation.

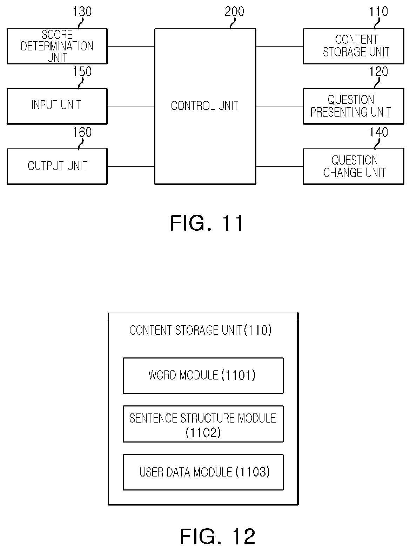

Accordingly, FIG. 11 specifically shows a detailed configuration of the personalized cognitive training device 400 according to an exemplary embodiment of the present disclosure.

As shown in FIG. 11, the personalized cognitive training device performing the plurality of cognitive training may include: a content storage unit 410 classifying and storing a content used for presenting a question for each level or item; a question presenting unit 420 presenting the corresponding question for the cognitive training in consideration of the user data and the brain region to be activated through the cognitive training; a score determination unit 430 determining a range of scores acquired from the questions presented by the question presenting unit 420 or determining whether to maintain the scores; and a question change unit 440 changing the question level or the question pool based on a result of the score determination unit 430. Here, the question change unit 440 may include a level adjustment module 1401 adjusting the question level to maintain a predetermined optimal score range, and a pool change module1402 changing the level or item of the content storage unit 410 to change the question pool when a cognitive-training result score of a current round remains the same as the cognitive-training result score of a previous round even though question difficulty is changed.

In detail, the pool change module 1402 may change a question type of a next round when the level adjustment module 1401 changes the question difficulty for the cognitive-training result score of the next round to be within the predetermined optimal score range, or when the cognitive-training result score of the current round remains the same as the cognitive-training result score of the previous round even though the question difficulty is changed for the cognitive-training result score of the next round to be within the predetermined optimal score range.

According to an exemplary embodiment of the present disclosure, the predetermined optimal score range may include scores of 85 points or more and 95 points or less, which is predetermined by the user. It is important for the cognitive training device 400 to continuously stimulate the user brain. When keeping getting 400 points, the user may guess a correct answer too easily, and the user may have rather lower concentration and get drowsy, thus lowering the brain stimulation efficiency. On the other hand, when getting a low score of 4 to 50, the question may be too difficult for the user, and the user may thus have lower interest and concentration, thus lowering the brain stimulation efficiency. Accordingly, the score range of 85 points to 95 points may be set to continuously maintain a brain activation state, which is an example, and the claims are not limited thereto.

Accordingly, the question change unit 440 may change the question difficulty or the question pool to maintain the predetermined optimal score range in consideration of the user's cognitive-training result score output from the score determination unit 430.

In addition, when changing the question difficulty or the question pool in consideration of the user's cognitive-training result score, it is possible to present a question having a difficulty level or question pool appropriate for brain function improvement that is adjusted based on the user's dementia progression or brain state. However, it is impossible to present the question based on the user data such as the user personal data or the user history. When the question is presented without considering the user data, words or sentences that the user does not know may exist regardless of the dementia progression. Even in this case, the user may give an incorrect answer to the question, thus providing erroneous feedback on the dementia progression of the user.

Therefore, when determining the question difficulty or question pool of the next round, the question type may be changed to another type in consideration of the user data. Here, the user data may include at least one of the user's educational background, spoken language, gender, residence history, and occupation history.

That is, the question presenting unit 420 may determine the question type based on the brain region to be activated, the level adjustment module 1401 of the question change unit 440 may adjust the question difficulty in consideration of the user cognitive-training result score output from the score determination unit 430, and when the user's result score is not changed even though the pool change module 1402 of the question change unit 440 adjusts the question difficulty, the level or item of the content storage unit 410 may be changed for the question type to be changed to another type.

FIGS. 12 through 14 schematically show a configuration of the content storage unit 410 and a structure in which the content storage unit 410 classifies and stores words according to an exemplary embodiment of the present disclosure.

As shown in FIG. 12, the content storage unit 410 according to an exemplary embodiment of the present disclosure may include a cognitive training data base (DB) including: a word module 4101 including words presented in a word question; a sentence structure module 4102 classifying and storing words based on a sentence structure for the words to be presented in a sentence question; and a user data module 4103 storing the user data including at least one of the user personal data and the user treatment data.

The word module 4101 is a DB storing words representing objects or actions. The word module 4101 may provide words used by the sentence structure module 4102 to generate a sentence to activate Broca, or provide a word or like used to find a correct word or match a word to a photo to thus activate Werniche, or find a correct action or object name to activate the right DLFP.

In addition, the sentence structure module 4102 may classify and store words into subjects, predicates, objects, complements, or functional words, and extract the words from each classified item when generating sentences.

In particular, the sentence structure module 4102 according to an exemplary embodiment of the present disclosure may configure a module having a sentence structure corresponding to a Korean sentence in accordance with Korean language. However, the sentence structure module 4102 may be configured to have a sentence structure corresponding to sentences in accordance with a language of another country such as English, Japanese, or Chinese.

In addition, the user data module 4103 may store the user history data including at least one of the user's educational background, language, gender, residence, and occupation, or the user treatment data storing the user's cognitive training content or cognitive-training result score.

Accordingly, the user data module 4103 may include a user treatment data DB storing the user history data and the user treatment data.

For example, the user may speak a plurality of languages, for example, both Korean and English. In this case, in consideration of the user data, the sentence structure module 4102 may be provided as the plurality of sentence structure modules 4102, not only including a sentence structure module corresponding to a Korean sentence, but also including a sentence structure module corresponding to an English sentence, and the question difficulty may be adjusted or the question pool may be changed by using the above languages.

Alternatively, the user's educational background may have an influence on the knowledge that the user has. Accordingly, the word module 4101 may also include a major term used in a graduate school when the user graduates from the graduate school.

Alternatively, a user experience may be different for each gender, and the word module 4101 may thus consider and reflect a term which may be learned from such experience.

Alternatively, based on the user's residence history, a user who lives only in Seoul cannot understand a regional dialect word regardless of dementia. However, the word module 4101 may include even a word used only in the regional dialect and use this word to perform the cognitive training on a user who lives in a region where dialect is used.

Therefore, the content storage unit 410 may structuralize and store the words or the user data, used in the question present based on the classification criteria as described above. The word module 4101 or the sentence structure module 4102 may extract words or sentences in consideration of the user data received from the user data module 4103, and present personalized questions for each user that may continuously stimulate the user brain.

In particular, in the question types corresponding to the brain region, matching a word to a photo, finding a correct action name, and finding a correct object name may be particularly affected by an environment, and it is thus very important to present a question in consideration of the user data.

In addition, the question difficulty and the question pool, used in the cognitive training, and the user history data may correspond to each other and then be stored in the user data module 4103.It is thus possible to use accumulated user data as big data, thus reducing the time and cost required to present or change the personalized question, and derive the optimal score range having the highest brain stimulation efficiency from the corresponding user data.

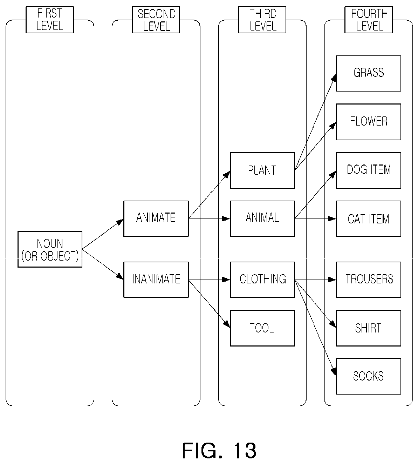

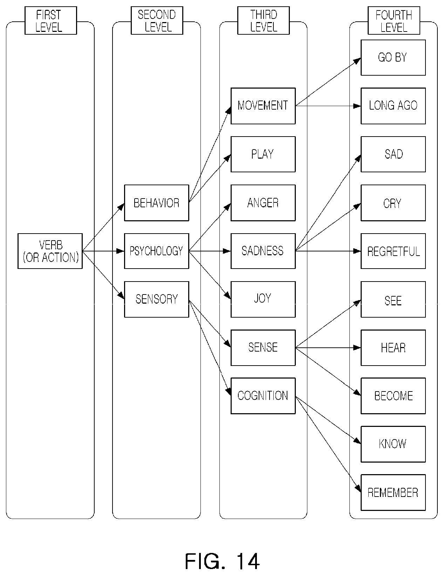

In detail, as shown in FIGS. 13 and 14, the content storage unit 410 according to an exemplary embodiment of the present disclosure may classify and store the words for each level or for each item of one level. Here, the levels may include first to nth levels, where n is a positive integer greater than or equal to 2.

In addition, according to an exemplary embodiment of the present disclosure, the first level of the content storage unit 410 may include a noun item and a verb item, and may include a lower concept word further subdivided from the item as the number of levels is further increased.

As shown in FIG. 13, the content storage unit 410 may store the words structuralized in the first to fourth levels (here, n=4). The first level may include one noun (or object) item, and the second level may include an animate item and an inanimate item, which are lower concepts subdivided from the noun (or object) item.

In addition, the third level may include a plant item and an animal item subdivided from the animate item of the second level, and a clothing item and a tool item subdivided from the inanimate item of the second level.

The fourth level may include grass and flower items subdivided from the plant item of the third level, dog and cat items subdivided from the animal item, andtrousers, shirts, and socks items subdivided from the clothing item of the third level.

For example, the question presenting unit 420 may present a question on whether an item corresponds to clothes or a tool while illustrating a photo of a millstone in the third level, or present a question on what an item is in the fourth level while illustrating the millstone and a sickle that embody the tools of the third level.

Although n is a number greater than or equal to 2, a level actually presented as a question may be presented at a level of the largest number or a level just before the largest number, and the remaining levels may be used to classify the question pool by structuring the levels and the items.

According to an exemplary embodiment of the present disclosure, when a user average score is continuously maintained constant out of the optimal range set by the user for the plurality of rounds even though the question difficulty is adjusted, the level or item of the content storage unit 410 may be changed for the question pool to be changed.

As a specific example, the content storage unit 410 shown in FIG. 13 may further include a fifth level, where n is set to 5. The fifth level may include sphagnum, oleander, kerchief, silage, and forsythia, sub-divided from a flower item of the fourth level. However, when a user 1 has a residence of Gangnam-gu, Seoul for life, and has no experience of seeing flowers, user 1 would not be able to know whether sphagnum, oleander, or kerchief are the correct words or words matching the photo regardless of dementia.

On the other hand, user 2 may be a botanist. In this case, even though living in Gangnam-gu, Seoul for all his/her life, user 2 would be able to know whether sphagnum, oleander, or kerchief, silage, or the like are the correct words, and would be able to choose the word matching the photo by looking at the corresponding photo. Therefore, when user 2 is unable to choose the above words due to severe dementia, feedback on user 2's dementia progression may be accurately received.

That is, even though a question having the same item at the same level is presented, whether or not a brain activation process in which the user thinks and derives an answer is performed may be different based on the user data.

Therefore, when user 1 continuously receives lower scores below the predetermined optimal score range, the pool change module 1402 may consider that the corresponding item does not have discernment factors enough to activate user 1's brain, lower the level from the fifth level to the fourth level of the content storage unit 410, and then change the item from the flower item to the dog item.

Alternatively, when user 1 continuously receives lower scores significantly below the predetermined optimal score range, the pool change module 1402 may consider that the corresponding item or an item similar thereto do not have the discernment factors enough to activate user 1's brain, lower the level from the fifth level to the third level of the content storage unit 410 by two levels, and then change the item from the plant item of the third level, which is a higher concept of the flower item, to the tool item of the third level.

On the other hand, when solving the question related to the flower item of the fourth level to continuously receive higher scores beyond the predetermined optimal score range, user 2 may have the reduced brain activation caused by the lower concentration due to excessively easy questions. Accordingly, the pool change module 1402 may increase the level and then present the sphagnum, oleander, or kerchief, silage items of the fifth level.

As shown in FIG. 14, the content storage unit 410 may structuralize the words into the first to fourth levels (here, n=4) and the store the same. The first level may include a verb (or action) item, and the second level may include a behavior item, a psychology item, and a perception item, which are lower concepts subdivided from the verb (or action) item.

In addition, the third level may include a movement item and a play item subdivided from the behavior item of the second level, an anger item, a sadness item, and a joy item subdivided from the psychology item of the second level, and a sense item and a cognition item subdivided from the perception item of the second level.

The fourth level may include items such as 'go by' and 'long ago' subdivided from the movement item of the third level, items such as 'sad' and 'cry' subdivided from the sadness item of the third level, and items such 'see,' 'hear,' and 'become,' subdivided from the sense item of the third level.

As a specific example, the user data of user 3 may include an office worker, a male, Seoul, and university graduate. In this case, if based on 'become' of the fourth level, it is impossible to determine whether the user's identity or status 'becomes' to be a new one or work 'becomes' too much for the user in a Gyeongsang-do dialect. For example, the above item 'become' may indicate the latter, 'becomes too much.' In this case, user 3 may select a sentence as an incorrect sentence when viewing the sentence described as 'The work becomes for him.' However, the user's answer is incorrect.

Therefore, when the average score is continuously maintained constant out of the optimal range set by the user for the plurality of rounds even though adjusting the question difficulty due to the repeated incorrect answers, the pool change module 4402 may lower the level of the content storage unit 410 to the third level for the question pool to be changed, and then change the item to the movement item of the third level to thus present a question.

Therefore, when determining the question pool, the pool change module 4402 may change the question type to another type in consideration of the user data.

According to an exemplary embodiment of the present disclosure, the pool change module 4402 may change the item by lowering the level of the word module for higher concept words to be presented when a low result score is maintained below the predetermined optimal score range, and change the item by increasing the level of the word module for lower concept words to be presented when a high result score is maintained beyond the predetermined optimal score range.

Alternatively, when the result score which is greatly different from the predetermined optimal score range is maintained, the item may be changed by lowering or increasing the level of the content storage unit 410 by the plurality of levels.

Meanwhile, the sentence structure module 4102 may include a subject item, a complement item, an object item, and a predicate item, and may further include a functional word item.

A similar 'sentence' is required to be presented, and a 'correct or incorrect sentence' is required to be presented to activate the Broca region. As a condition for forming a sentence, a predicate is required to be included, and a subject may be added to the predicate, the subject and an object may be added, the subject and a complement may be added, or the subject, the object and the complement may be added.

The question presenting unit 420 may change the question type or adjust the question difficulty by adding or deleting the subject, the object, and the complement, which are prefixes attached to the predicate, and form the correct or incorrect sentence by adjusting the existence and position of the predicate.

For example, the functional word item may include relative words such as adjectives, modifiers, and postpositional words, and independent words such as connective words, verbs, operational words, and interjections.

Meanwhile, the level adjustment module 4401 according to an exemplary embodiment of the present disclosure may give a priority to an item related to the user data received from the user data module 4103 and present a question from the question pool related to the input user data when lowering the question difficulty or the current cognitive training round is a first round.

As described above, the item related to the user data is a word that the user already encounters, and may also have an influence on the question difficulty. Therefore, in the first round in which the question difficulty is reduced, or the cognitive training is performed without previous data, it is possible to give a higher priority to the item related to the user data for the user to solve the question more easily.

In addition, the level adjustment module 4401 may perform the adjustment in which only a specific letter is increased or a total number of letters is reduced, for example, to lower the difficulty in the question of 'finding a specific letter.'

Alternatively, the level adjustment module 4401 may reduce syllables of words or the number of words that forms a sentence to lower the difficulty in a question related to a sentence and a word, such as selecting a similar sentence, selecting a correct or incorrect sentence, finding a correct word or finding a word matching a photo.

In addition, when adjusting the question difficulty, the level adjustment module 4401 may adjust the question difficulty by one level based on the result score, and may adjust the question difficulty by a plurality of levels when the result score is too far out of the optimal score range.

A personalized cognitive training method performed by the personalized cognitive training device performing the plurality of cognitive training according to an exemplary embodiment of the present disclosure may include: presenting a predetermined type of question based on a brain region to be trained when the user data is input to the device; determining a cognitive-training result score based on user feedback on the presented question; and determining the question difficulty or question pool of the next round based on the cognitive-training result score of the current round.

Here, in the determining the question difficulty or question pool of the next round, the question difficulty may be changed for the cognitive-training result score of the next round to be within the predetermined optimal score range, or the question type of the next round may be changed when the cognitive-training result score of the current round remains the same as the cognitive-training result score of the previous round even though the question difficulty is changed.

Here, the 'round' refers to a cognitive training routine repeated at a predetermined period, which may be predetermined by the user. For example, when determined in units of a week, the 'round' may be performed on the basis of a first week, a second week, ..., and an Nth week.

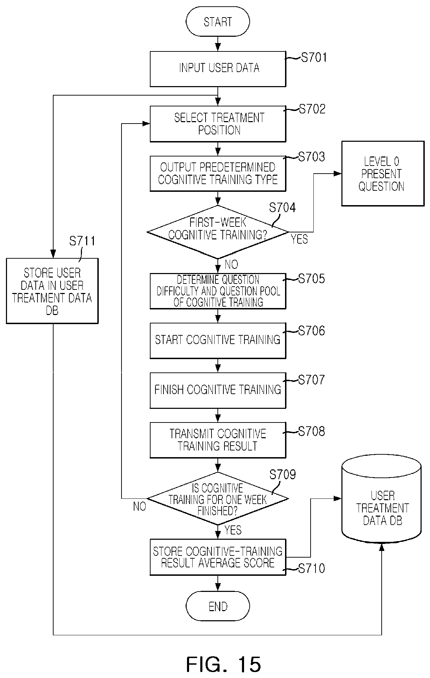

FIG. 15 is a flow chart of the personalized cognitive training method according to an exemplary embodiment of the present disclosure. As shown in FIG. 15, user data may be input to the personalized cognitive training device 400 (S701), the treatment position may be selected from the brain regions based on a predetermined schedule (S702), and a predetermined cognitive training type corresponding to the brain region may be output (S703). This type may be the same as the cognitive training type corresponding to the brain region described above.

In addition, according to an exemplary embodiment of the present disclosure, when the round is performed in units of a week, according to the personalized cognitive training method of performing the plurality of cognitive trainings, the training may be performed for a total of 4 weeks in units of a week.

Here, when the training is a first-week cognitive training (Yes in S704), as no cognitive-training result score of the previous round exists, and a question of the item related to the user data may thus be presented using the user data input to the device. In addition, the easiest question having the difficulty of a level zero may be presented.

On the other hand, when the training is not the first-week cognitive training (No in S704), the question pool and difficulty may be determined using the user data input to the device and the cognitive-training result score of the previous round (S705).

The cognitive training may start based on the cognitive training type and the question difficulty and pool of the cognitive training that are determined above (S706), and when the cognitive training is finished after the cognitive training is performed based on the predetermined method (S707), the cognitive training result may be transmitted to the user (S708).

When the cognitive training for the predetermined period of one week is finished (Yes in S709), the average score may be calculated for the cognitive-training result scores for the predetermined period of time, and the cognitive-training result average score may be stored (S710). Here, the result average may be stored in the user treatment data DB, which may be included in the user data module 4103.

Meanwhile, the user data input in S701 may also be stored in the user treatment data DB (S711).

Meanwhile, in S703, according to another exemplary embodiment of the present disclosure, when the plurality of brain regions are simultaneously trained during each cognitive training, a question in which the predetermined type of questions are mixed based on the brain region may be presented. That is, the plurality of brain regions may receive the cognitive training simultaneously, and each brain region may receive the cognitive training individually, and the question type may be different based thereon.

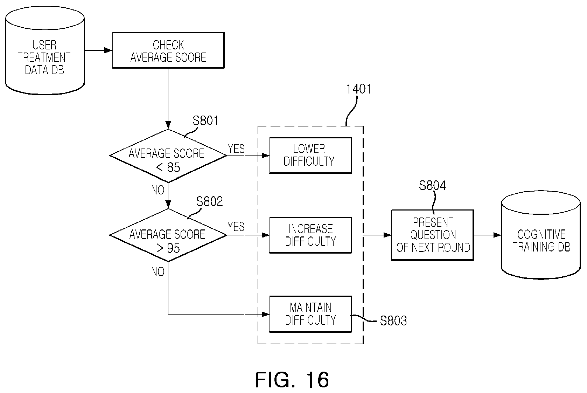

FIGS. 16 through 19 are flow charts illustrating the level adjustment or question pool adjustment of the personalized cognitive training method according to an exemplary embodiment of the present disclosure.

After performing the cognitive training and acquiring and storing the result average score as described with reference to FIG. 15 (S710), as shown in FIG. 16, the level adjustment module 4401 may check the result average score stored in the user treatment data DB, and adjusting the question difficulty of the next round based thereon.

For example, it is assumed that the round is performed in units of a week, and the optimal score range predetermined by the user is between 85 and 95 points.

The level adjustment module 4401 may check whether the average score stored in the user treatment data DB falls within the optimal score range.

In detail, the level adjustment module 4401 may lower the question difficulty when the average score is less than 85 points (Yes in S801), and increase the question difficulty when the average score is more than 95 points (Yes in S802). On the other hand, the level adjustment module 4401 may maintain the question difficulty when the average score is between 85 and 95 points within the optimal score range (No in S801 & No in S802).

That is, the level adjustment module 4401 may adjust the question difficulty based on the average score (S803).

Next, the level adjustment module 4401 may present a question of the next week based on the determined question difficulty (S804), and store the presented question in the cognitive training DB.

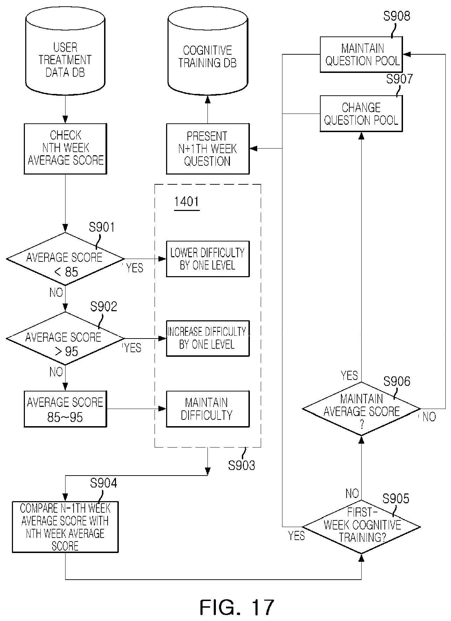

In addition, as shown in FIG. 17, the level adjustment module 4401 may adjust the level based on the current average score, and the pool change module 4402 may then change the question pool based on a change degree between the average score of a previous week and the average score of a current week.

As shown in FIG. 17, steps S901 to S903 performed after checking the Nth week average score may be different in order from the steps S801 to S803 shown in FIG. 16. However, the steps S901 to S903 may be the same as the steps S801 to S803 in checking whether the average score falls within the predetermined optimal score range.

The level adjustment module 4401 may determine the question difficulty of the next round (i.e., N+1th week) (S903), then compare an N-1th week average score with the Nth week average score (S904), and determine whether the pool change module 4402 changes the question pool.

The question pool may be changed (S907) when the average score is maintained (Yes in S906) based on the comparison of the N-1th week average score with the Nth week average score (S904), and the question pool may be maintained (S908) when there is a change in the average score (No in S906) by changing the question difficulty.

However, the above process may be performed when N corresponds to 2 or more (No in S905). When N corresponds to the first week, i.e., N = 1 (Yes in S905), there is no previous-week average score, and a second week (i.e., N+1th week) question may be immediately presented and stored without changing the question pool.

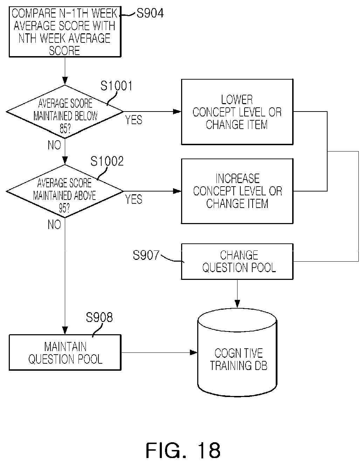

The question pool change flow shown in FIG. 17 is specifically shown in FIG. 18 illustrating how the pool change module 4402 adjusts the question pool based on the cognitive-training result average score.

As shown in FIG. 18, when the N-1th week average score and the Nth week average score are compared (S904) and the average score is maintained below 85 points (Yes in S4001), a word concept level of the content storage unit 410 may be lower or the item may be changed.

In addition, when the average score is maintained above 95 points (Yes in S4002), the word concept level of the content storage unit 410 may be increased or the item may be changed.

That is, the question pool may be changed (S907) when the average score is maintained below 85 points or above 95 points, and the question pool may be maintained (S908) when the average score is maintained within the predetermined optimal range of 85 points or more and 95 points or less.

The pool change module 4402 may determine the question pool through the above-mentioned process, store the same in the cognitive training DB, and then use the stored question pool in the cognitive training of the next round.

Therefore, the personalized cognitive training device performing the plurality of cognitive training according to an exemplary embodiment of the present disclosure may present the predetermined type of question based on the user data input to the device and the brain region to be trained, determine the cognitive-training result score based on a user answer to the presented question, change the question difficulty for the cognitive-training result score of the next round to be within the predetermined optimal score range based on the cognitive-training result score of the current round, or change the question type of the next round when the cognitive-training result score of the current round remains the same as the cognitive-training result score of the previous round even when changing the question difficulty.

In addition, in detail, the personalized cognitive training device according to an exemplary embodiment of the present disclosure may present a question from the question pool that is highly related to the user data when both the cognitive-training result score of the previous round and the cognitive-training result score of the current round that is acquired by changing the question difficulty are below the predetermined optimal score range, and present a question from the question pool that are not related to the user data,when both the scores exceed the predetermined optimal score range. The question pool and difficulty may be determined in consideration of the user data.

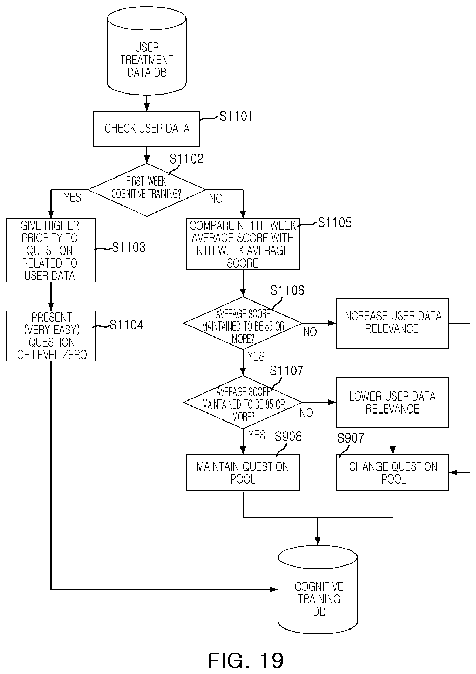

FIG. 19 is a flow chart illustrating the level adjustment and question pool adjustment of the personalized cognitive training method, performed in consideration of the user data, according to an exemplary embodiment of the present disclosure.

According to an exemplary embodiment of the present disclosure, the question type may be changed to another type in consideration of the user data, when the question pool is determined in the determining of the question difficulty or question pool of the next round based on the cognitive-training result score of the current round.

Here, according to an exemplary embodiment of the present disclosure, the user data may include at least one of the user's educational background, spoken language, gender, residence history, and occupation history.

That is, when determining the question pool, the pool change module 4402 may determine the pool in consideration of the user data.

In detail, as shown in FIG. 19, the input user data may be checked in the user treatment data DB included in the user data module 4103 (S1101).

When the cognitive training is not the first-week cognitive training (No in S1102), the N-1th week average score and the Nth week average score may be compared with each other (S1105). Here, the question pool may be changed to another question pool having high relevance to the user data when the result average score is less than 85 points (No in S1106), and changed to another question pool having low relevance to the user data (S907) when the result average score exceeds 95 points (Yes in S1106 & No in S1107).

On the other hand, the question pool may be maintained regardless of the user data when the result average score is 85 points or more and 95 points or less.

That is, when changing the question pool, it is possible to change the question pool by determining the priority of the question pool in consideration of the user data, as well as simply changing to another question type.

The user may have more data on the item related to the user data and answer questions more easily, and the question pool may thus be changed in consideration of the user data.

Meanwhile, when the cognitive training is the first-week cognitive training (Yes in S1102), there is no previous-week average score. Therefore, higher priority may be given to the question type related to the user data (S1103) to perform a very easy level of cognitive training in evaluating the user state. Here, a question of the level zero in the corresponding question pool may be presented (S1104) to present a low-level question of the question pool that is highly related to the user.

In this way, similar to changing the question type, when performing the cognitive training for the first time, it is possible to check the user state by lowering the level of the item related to the user data, thus making it easier for the user to answer the question.

Therefore, according to another exemplary embodiment of the present disclosure, in the changing of the level or item of the content storage unit 410, the priority for presenting the question of the items related to the user data may be adjusted using the user data input to the device 400 and the cognitive-training result score of the current round.

In detail, according to another exemplary embodiment of the present disclosure, the level of the content storage unit 410 may include the lower concept words further subdivided from the item as the number of levels is further increased, the level may be lower while giving the priority to an item having high relevance to the user data when the average score is continuously maintained below the optimal range set by the user for the plurality of rounds, and the level may be increased while giving the priority to an item having low relevance to the user data when the average score is continuously maintained beyond the optimal range set by the user for the plurality of rounds.

In addition, when the current cognitive training round is the first round, the question pool may be determined by giving the priority to the item highly related to the user data, the question pool of the next round may be also maintained as the question pool of the first round, and the question difficulty of the current cognitive training may be presented to the minimum.

In this way, even though the question difficulty is continuously adjusted, the cognitive-training result score of the user performing the cognitive training may remain very low or very high, thus preventing proper brain stimulation. In this case, the user cannot perform further cognitive training and may be excluded from the dementia treatment.

Through the personalized cognitive training of the present disclosure, the user may perform the cognitive training relevant to the user's own history and treatment data, and any user may thus receive a dementia treatment effect through the cognitive training question relevant to the user, and an existing user may have an improved treatment effect.

According to an exemplary embodiment of the present disclosure, the brain region to be trained may be at least one region of Broca, Werniche, L-DLPFC, R-DLPFC, L-PSAC and R-PSAC, and a question in which the pre-determined type of questions based on the brain region are mixes may be presented during each cognitive training.

In addition, when training the plurality of brain regions simultaneously during each cognitive training, a new type of question in which the predetermined question types are mixed may be presented.

According to an exemplary embodiment of the present disclosure, the words used in the cognitive training may be classified for each level or for each item of one level and then stored. Here, in the personalized cognitive training device 400 including the content storage unit 410, where the levels includes the first level to the nth level, and n is a positive integer greater than or equal to 2, and the levels include a level in which the average score is calculated through the input answers to the plurality of types of questions submitted to activate each brain region for a predetermined period for each round, and a level in which the level or item of the content storage unit 410 is changed for the question pool to be changed when the average score is continuously maintained constant out of the optimal range set by the user for the plurality of rounds even when adjusting the question difficulty.

According to an exemplary embodiment of the present disclosure, the first level of the content storage unit 410 may include the noun item and the verb item, and may include the lower concept word further subdivided from the item as the number of levels is further increased.

In addition, according to an exemplary embodiment of the present disclosure,when the current cognitive training round is the first round, a question may be presented from the question pool related to the input user data. The question difficulty may be lower by presenting a question of the item highly related to the user.

In addition, the level adjustment module 4401 may change the level to adjust the question difficulty before the pool change module 4402 changes the pool. According to an exemplary embodiment of the present disclosure, it is possible to adjust the number of words extracted from the DB, a length of sentences, the number of colors, or the number of figures based on the question difficulty.

In addition, the level adjustment module 4401 may give the priority to the item related to the user data received from the user data module 4103 and present a question from the question pool related to the input user data when lowering the question difficulty or the current cognitive training round is the first round.

In detail, the level adjustment module 4401 according to an exemplary embodiment of the present disclosure may maintain the question difficulty of the next round when the cognitive-training result score of the current round is within the predetermined optimal score range, reduce the question difficulty of the next round when the cognitive-training result score of the current round is below the predetermined optimal score range, and increase question difficulty when the cognitive-training result score of the current round is beyond the predetermined optimal score range.

Meanwhile, the cognitive training device 400 according to an exemplary embodiment of the present disclosure may include an input unit 450 receiving an answer input by the user to a question and an output unit 460 outputting a question for the question to be provided to the user. Here, the input unit 450 and the output unit 460 may be included in the user interface 401 of FIGS. 4 and 8.

The output unit 460 may be a display module, and the input unit 450 may be an input interface which may receive an answer input by the user such as a tablet personal computer (PC), laptop PC, or a desktop computer and provide the same to a control unit 470 or may be a device including a text to speech (TTS) program automatically storing a user's voice answer in a computer.

Alternatively, according to another exemplary embodiment of the present disclosure, even though the presented question is provided to the user through the output unit 460, the answer to the question may be directly input by the user in the form of a direct answer to the medical personnel such as a nurse. The reason is that a dementia user may have poor motor skills, and the user answer may not be received properly when the answer is processed using a machine.

In addition, in detail, according to an exemplary embodiment of the present disclosure, the word module 4101 may include at least one item of words, colors, figures, and photos, the sentence structure module 4102 may include at least one item of subjects, predicates, objects, and complements, and the user data module 4103 may include at least one item of the user's educational background, language, gender, residence, and occupation.

That is, colors, figures, and photos stored in the word module 4101 may also be presented as corresponding ones based on the question difficulty. For example, the number of colors or shapes may be adjusted, and a photo related to a foreign country may be presented as a question to lower the question difficulty based on the user data module 4103 because a user who lived abroad is not unfamiliar with a foreign culture.

In addition, the question presenting unit 420 may output a question of a corresponding type by using at least one of the brain region to be stimulated, the question difficulty determined by the level adjustment module 4401, and the pool changed by the pool change module 4402 and using the data stored in the word module 4101, the sentence structure module 4102, and the user data module 4103.

In this way, the personalized cognitive training may be provided to all users with no user excluded from the dementia treatment, and the brain region may be stimulated more accurately and efficiently through the cognitive training question reflecting the user data such as each user's history, thus improving the effect of the dementia treatment. In addition, accuracy of the brain region stimulation may be further increased by adjusting the question pool as well as adjusting the question difficulty.

In addition, the database may be easily expanded because the words used in a question are classified and stored based on the level or item. It is possible to easily add words to the existing DB, and its extendability may thus be secured when new terms or terms referring to new things appear as time goes by, and new words need to be added.

In particular, the content storage unit 410 of the present disclosure may be continuously updated by receiving not only a word DB collected directly by securing the extendability but also a word DB collected by a third party such as Google search or Wikipedia.

Accordingly, the content storage unit 400 according to an exemplary embodiment of the present disclosure may be linked with a web server, receive a new content from the web server, and update a content used for presenting a question.

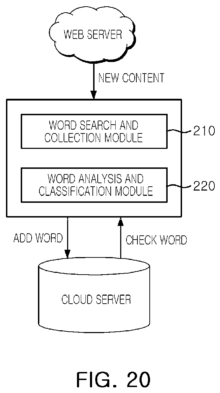

Hereinafter, FIGS. 19 through 22 show the control unit 470 searching for, collecting, and analyzing or classifying new words.

In detail, as shown in FIG. 19, the control unit 470 according to an exemplary embodiment of the present disclosure may include a word search and collection module 210 linked with a server to update the content used for presenting a question, and searching for and collecting the new content from the server; and a word analysis and classification module 220 analyzing the new content and classifying the same for each existing level or item.

As shown in FIG. 19, the control unit 470 may search for or collect a content including at least one of sentences, words, and images from a web server, and the web server may include the database collected by the third party.

The searched or collected content may be classified for each level or item based on the classification method of the content storage unit 410 of the present disclosure, and immediately stored in the content storage unit 410. Alternatively, the analyzed or classified content may be stored in the cloud server for each level or item. It is possible to secure the extendability or manageability of the content by storing the cognitive training DB in the cloud server.

In addition, according to an exemplary embodiment of the present disclosure, the word analysis and classification module 220 may store a corresponding word as a lower level of a higher concept word or a higher level of a lower concept word when the higher concept word or lower concept word related to the searched content exists in the content storage unit 410, and the word search and collection module 210 may search for and collect the higher concept word or lower concept word related to the searched content when no higher or lower concept word related to the searched content exists in the content storage unit 410.

In addition, the word analysis and classification module 220 according to an exemplary embodiment of the present disclosure may analyze or classify the content by using an artificial intelligence. Here, the content may include at least one of words, sentences, and images.

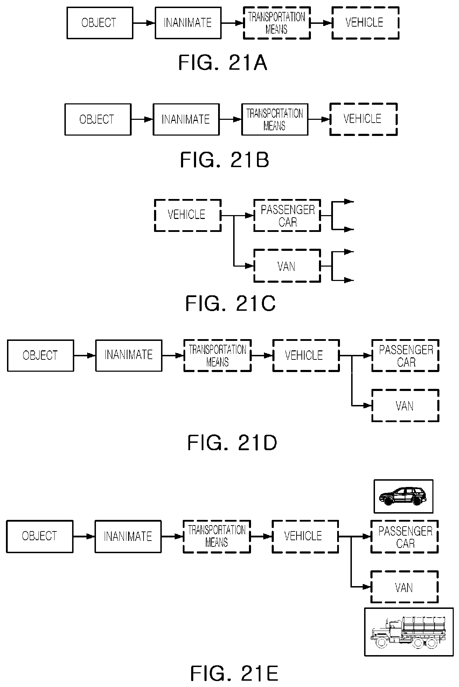

In detail, as shown in FIG. 21, the content storage unit 410 may store 'objects' as the first level, 'inanimate objects' as the second level, 'transportation means' as the third level, and 'vehicle' as the fourth level, and classify the corresponding item from a higher concept to lower concept for each level.

When searching for the word 'vehicle' as the new content from the web server and failing to find the same in the cloud server, the word search and collection module 210 may collect and store the corresponding word in the cloud server or the content storage unit 410.

Here, as shown in FIG. 21A, when a higher concept of the word 'vehicle' exists and this higher concept does not exist in the content storage unit 410, the word search and collection module 210 may search for 'transportation means,' which is the higher concept of the word 'vehicle,' and the word analysis and classification module 220 may classify the corresponding word into transportation means-vehicle and store the same for each level.

Alternatively, as shown in FIG. 21B, when a higher level concept of the word 'vehicle' exists and this higher level concept exists in the content storage unit 410, the 'vehicle' may be stored as a lower concept in the next level of the higher level concept 'transportation means.'

Meanwhile, as shown in FIG. 21C, when a lower concept of the word 'vehicle' which is the new content exists, a lower concept word related to the 'vehicle' may be searched and collected by the word search and collection module 210, and then classified and stored as vehicle-passenger car, van, or the like. In addition, a lower concept of the passenger car or the van may be also searched and collected, and stored as the next level of the passenger car and the van. The lower concept words may overlap each other based on the level. However, even the overlapping lower concept words may have different levels of related higher concepts.

In addition, as shown in FIG. 21D, the content may be classified in the above-described manner and stored in a total of 5 levels: object-inanimate object-transportation means-vehicle-passenger car or van. Here, not only the words but also the levels and related items of the words may be stored in the content storage unit 410 or stored in the DB in the cloud server.

In addition, as shown in FIG. 21E,images such as photos, pictures, and figures that are related to words may be also matched and stored in the content storage unit 410, orthe DB in the cloud server. In this way, such data may be used for a question type such as finding words matching the photo.

Here, an image matching a word may be required when performing the 'finding a word matching a photo' cognitive training to stimulate the Werniche region based on newly added words or the 'finding a correct object name' cognitive training to stimulate the R-DLPSC region.

Therefore, images may be classified using an artificial intelligence. For example, images of trucks and passenger cars may be retrieved and collected through the artificial intelligence. Here, the image of the truck may be classified and stored by matching a word 'truck' stored in the fifth level, and the image of the 'passenger car' may be classified and stored by matching a word 'passenger car' stored in the fifth level. The artificial intelligence may be trained using a convolution neural network (CNN) or the like.

For another exemplary embodiment, words retrieved and collected using the artificial intelligence may be classified and stored.

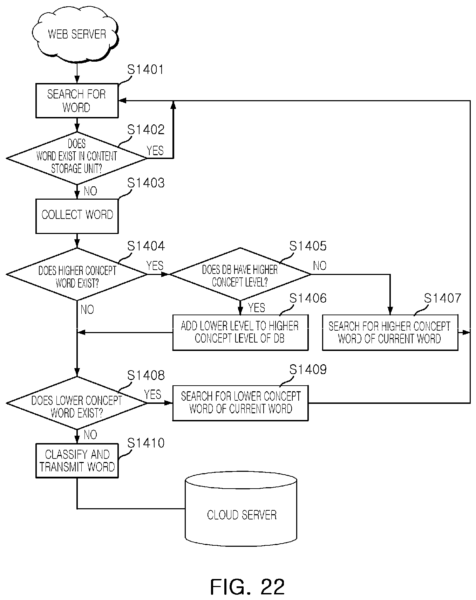

Therefore, as shown in FIG. 22, the word search and collection module 210 may search the web server for the word 'vehicle' as the new content (S1401), and may collect the word 'vehicle' (S1403) when the word is not found in the content storage unit 410 as a new word (No in S1402). When the word exists in the content storage unit 410 (Yes in S1402), the word search and collection module 210 may search for the new content again (S1401).

The word analysis and classification module 220 may then analyze the word 'vehicle,' determine whether a higher concept word of the word exists (S1404), and determine whether a lower concept word of the word exists (S1408).

In detail, when a higher concept word of the word 'vehicle' exists (Yes in S1404), the word analysis and classification module 220 may determine whether the higher concept word, for example, the word 'transportation means' exists in the DB of the cloud server or the content storage unit 410 (S1405), and when the higher concept word exists (Yes in S1405), the word analysis and classification module 220 may add the lower concept word 'vehicle' to a lower level of a level of the higher concept word 'transportation means' in the DB of the cloud server or the content storage unit 410.

On the other hand, when no higher concept word does exists in the DB of the cloud server or the content storage unit 410 (No in S1405), the word search and collection module 210 may search for the higher concept word of the current word 'vehicle' (S1407) to collect the words, and store these words for each level by relating the higher concept word to the lower concept word.

In addition, when the lower concept word of the word 'vehicle' exists (Yes in S1408), the word search and collection module 210 may further search for the lower concept words, the 'van' and the 'passenger car' of the new word 'vehicle' (S1409), and store the new word 'vehicle' and its lower concept words the 'van' and the 'passenger car' for each level in the DB of the cloud server or the content storage unit 410.

That is, it may be determined whether a higher concept word of the word exists (S1404), it may be determined whether a lower concept word exists (S1408), and a new content may be classified through the above-described classification and analysis, and the classified new content may be transmitted to be stored in the DB of the cloud server or the content storage unit 410 (S1410).

Accordingly, the personalized cognitive training method according to an exemplary embodiment of the present disclosure, performed by the personalized cognitive training device performing the plurality of cognitive training, may include: inputting the user data to the device and presenting the predetermined type of question based on the brain region to be trained; determining the cognitive-training result score based on the user feedback on the presented question; anddetermining the question of the next round based on the cognitive-training result score of the current round, wherein in the determining of the question of the next round, the question type of the next round is changed based on the cognitive-training result score.

In addition, in the determining of the question of the next round, the question type may be changed when a change in the cognitive-training result score is within a predetermined range even though the question difficulty is changed while at least one of the question difficulty and the question type is changed or maintained based on the cognitive-training result score. In detail, the question pool may be changed to another question pool including the question type related to the user data, or another question pool including the question type unrelated to the user data for the cognitive-training result score of the next round to be within the predetermined optimal score range.

Alternatively, the level or item of the content storage unit may be changed so that the question pool is changed. Here, the level or item may be changed by adjusting the priority for presenting the question of the item related to the user data by using the user data input to the device and the cognitive-training result score of the current round. Through the input data, it is possible to classify the related item and the unrelated item, and adjust the question pool.

The magnetic stimulation method or the personalized cognitive training method according to an exemplary embodiment of the present disclosure described above may be produced as a program to be executed on a computer and stored in a computer-readable recording medium. Examples of the computer-readable recording medium may include a read only memory (ROM), a random access memory (RAM), a compact disk read only memory (CD-ROM), a magnetic tape, a floppy disk, an optical data storage device, or the like. In addition, the computer-readable recording medium may be distributed over computer systems connected with each other through a network, and a computer-readable code may thus be stored and executed in a distributed manner. In addition, the functional programs, codes and code segments for implementing the method may be easily inferred by programmers in the art to which the present disclosure pertains.

In addition, in describing the present disclosure, the '~ module' may be implemented in various ways, for example, may be implemented in a processor, a program instruction performed by the processor, a software module, a microcode, a computer program product, a logic circuit, an application-specific integrated circuit, a firmware, or the like.

In addition, in describing the present disclosure, the 'memory' may include both the external and internal store devices such as a universal serial bus (USB), and the cloud server may be used as the memory.

The present disclosure is not limited by the exemplary embodiments described above and the accompanying drawings. The scope of the present disclosure is limited by the appended claims, and it is apparent to those skilled in the art that various substitutions, modifications, and changes may also fall within the scope of the present disclosure without departing from the spirit of the present disclosure disclosed in the claims.

### [SEQUENCE LISTING FREE TEXT]

| | | | |
|---|---|---|---|
| 100: | MAGNETIC FIELD GENERATION DEVICE | 110: | CONTROL UNIT |
| 1101: | MAGNETIC FIELD CHANGE MODULE | 1102: | MOTOR THRESHOLD MEASUREMENT MODULE |
| 120, 120A, OR 120B: | POWER MODULE | 121: | POWER CONVERSION MODULE |
| 130: | COMMUNICATIONS MODULE | 140: | COOLING MODULE |
| 141: | COOLING UNIT | 142: | TEMPERATURE MONITORING UNIT |
| 150: | USER INTERFACE | 151: | INPUT MODULE |
| 152: | OUTPUT MODULE | 160, 160A, 160B: | COIL |
| 200: | BRAIN NAVIGATION DEVICE | 210: | MRI DATA MEMORY |
| 220: | MAPPING MODULE | 221: | CAMERA UNIT |
| 222: | IR LED UNIT | 230: | LASER SCANNER |
| 240: | LASER POINTER | 250: | COMMUNICATIONS MODULE |
| 260: | CONTROL UNIT | 270: | POWER MODULE |
| 300: | INTEGRATED OPERATOR | 301: | USER INTERFACE |
| 302: | SECOND MEMORY | 400: | PERSONALIZED COGNITIVE TRAINING DEVICE |
| 401: | USER INTERFACE | 402: | FIRST MEMORY |
| 410: | CONTENT STORAGE UNIT | 4101: | WORD MODULE |
| 4102: | SENTENCE STRUCTURE MODULE | 4103: | USER DATA MODULE |
| 420: | QUESTION PRESENTING UNIT | 430: | SCORE DETERMINATION UNIT |
| 440: | QUESTION CHANGE UNIT | 450: | INPUT UNIT |
| 460: | OUTPUT UNIT | 470: | CONTROL UNIT |
| 4401: | LEVEL ADJUSTMENT MODULE | 4402: | POOL CHANGE MODULE |

## Claims

1. A magnetic stimulation system comprising a magnetic field generation device, wherein the magnetic field generation device (100) includes:
a control unit (110) configured to determine a corresponding magnetic field in consideration of user data, including a motor threshold and a brain region to be stimulated;
a power module (120) connected to a coil (160) configured to generate a magnetic field and supplying power for the coil to generate the determined magnetic field; and
a motor threshold measurement sensor, and
the control unit includes:
a motor threshold measurement module (1102) configured to measure the motor threshold of a user by using a magnetic field change module (1101) by stepwise adjusting the magnetic field intensity by the magnetic field change module, and measure the user motor threshold by increasing magnetic field frequency by a predetermined ratio R1 and then stepwise adjusting the magnetic field intensity again when the magnetic field intensity reaches the maximum magnetic field output, wherein the measuring of the motor threshold of the user includes:
a first level of performing magnetic field stimulation on a motor cortex region of the user by the coil to check whether a change in finger movement or a change in user motility occurs using the motor threshold measurement sensor; a second level of increasing the magnetic field intensity by the predetermined ratio R1 until a change in the finger movement or motility occurs when no change finger movement or no change in the motility occurs; and a third level of increasing the magnetic field frequency when the magnetic field intensity reaches a predetermined maximum magnetic field output, and returning to the second level; and
when the change in finger movement or the change in the motility occurs, the motor threshold measurement module is configured to lower the magnetic field intensity at a predetermined ratio R2 that is smaller than the R1 by using the magnetic field change module and output the lowered magnetic field intensity, and when the finger movement or the change in the motility occurs no longer, the motor threshold measurement module is configured to determine the magnetic field intensity as the motor threshold;
the magnetic field change module configured to control the output magnetic field by adjusting a magnetic field-related parameter, and
the magnetic field change module configured to determine the magnetic field output based on the measured motor threshold, and a distance of a treatment position to the brain region to be stimulated, and a user brain state including at least one of user brain shape, brain size, and head shape, acquired using a received MRI image of the user.

2. The system of claim 1, wherein the magnetic field change module performs magnetic field stimulation by driving the coil with the magnetic field intensity determined by calculating the user motor threshold measured at the predetermined ratio R1 based on the brain region to be stimulated and the user brain state.

3. The system of claim 1, wherein the magnetic field-related parameter includes at least one of frequency, time, period, pulse width, and intensity of the magnetic field.

4. The system of claim 1, further comprising a brain navigation device detecting a position of the brain region to be stimulated by analyzing the MRI image of the user and guiding a position of the magnetic field generation device.

5. The system of claim 4, further comprising:
an MRI data memory configured to receive and store the MRI image of the user and data on the position of the brain region to be stimulated; and
a mapping module configured to capture an actual user head image in real time by using a camera, and mapping the actual head image captured in real time with the MRI image of the user brain displaying the data on the position of the brain region to be stimulated that is stored in the MRI data memory.

6. The system of claim 1, further comprising a cognitive training device linked to the magnetic field generation device to perform cognitive training by presenting a predetermined question corresponding to the brain region to activate a brain region stimulated by the magnetic field generation device after the brain is stimulated by the magnetic field generation device.

7. The system of claim 6, further comprising an integrated operator communicating with the magnetic field generation device and the cognitive training device, and simultaneously or alternately driving the magnetic field generation device and the cognitive training device.

8. The system of claim 7, wherein the integrated operator or the cognitive training device further includes at least one memory, and the memory is a database (DB) configured in a cloud server.

9. The system of claim 5, further comprising a robot arm receiving coordinates of the position of the brain region to be stimulated in the actual head that is acquired by the mapping module and moving the coil to correspond to the position coordinates; and
a laser scanner scanning the user head to detect a head position corresponding to the position of the brain region to be stimulated,
wherein the laser scanner scans the actual head to acquire data on a position of the actual head, and transmits data on the head position to the robot arm.

## Patentansprüche

1. Ein Magnetstimulationssystem, das eine Magnetfelderzeugungseinrichtung aufweist, wobei die Magnetfelderzeugungseinrichtung (100) Folgendes umfasst:
eine Steuereinheit (110), eingerichtet zum Bestimmen eines entsprechenden Magnetfelds unter Berücksichtigung von Benutzerdaten, einschließlich einer motorischen Schwelle und einer zu stimulierenden Gehirnregion;
ein Leistungsmodul (120), das mit einer Spule (160) verbunden ist, die eingerichtet zum Erzeugen eines Magnetfelds ist, und
das Leistung für die Spule zum Erzeugen des bestimmten Magnetfelds liefert; und
einen Sensor zum Messen der motorischen Schwelle,
wobei die Steuereinheit Folgendes umfasst:
ein Modul zum Messen der motorischen Schwelle (1102), eingerichtet zum Messen der motorischen Schwelle eines Benutzers unter Verwendung eines Magnetfeldänderungsmoduls (1101) durch schrittweises Einstellen der Magnetfeldintensität mittels des Magnetfeldänderungsmoduls und zum Messen der motorischen Schwelle des Benutzers durch Erhöhen der Magnetfeldfrequenz um ein vorbestimmtes Verhältnis R1 und anschließendes erneutes schrittweises Einstellen der Magnetfeldintensität, wenn die Magnetfeldintensität die maximale Magnetfeldausgabe erreicht, wobei das Messen der motorischen Schwelle des Benutzers Folgendes umfasst:
eine erste Stufe des Durchführens einer Magnetfeldstimulation an einer motorischen Kortexregion des Benutzers mittels der Spule zum Überprüfen, ob unter Verwendung des Sensors zum Messen der motorischen Schwelle eine Änderung der Fingerbewegung oder eine Änderung der Motorik des Benutzers auftritt; eine zweite Stufe des Erhöhens der Magnetfeldintensität um das vorbestimmte Verhältnis R1, bis eine Änderung der Fingerbewegung oder der Motorik auftritt, wenn keine Änderung der Fingerbewegung oder keine Änderung der Motorik auftritt; und eine dritte Stufe des Erhöhens der Magnetfeldfrequenz, wenn die Magnetfeldintensität eine vorbestimmte maximale Magnetfeldausgabe erreicht, und des Zurückkehrens zu der zweiten Stufe; und wenn die Änderung der Fingerbewegung oder die Änderung der Motorik auftritt, ist das Modul zum Messen der motorischen Schwelle eingerichtet zum Verringern der Magnetfeldintensität um ein vorbestimmtes Verhältnis R2, das kleiner als R1 ist, unter Verwendung des Magnetfeldänderungsmoduls und zum Ausgeben der verringerten Magnetfeldintensität, und wenn die Fingerbewegung oder die Änderung der Motorik nicht länger auftritt, ist das Modul zum Messen der motorischen Schwelle eingerichtet zum Bestimmen der Magnetfeldintensität als motorische Schwelle;
das Magnetfeldänderungsmodul eingerichtet zum Steuern des Ausgangsmagnetfelds durch Einstellen eines magnetfeldbezogenen Parameters, und
das Magnetfeldänderungsmodul eingerichtet zum Bestimmen der Magnetfeldausgabe auf Grundlage der gemessenen motorischen Schwelle, eines Abstands einer Behandlungsposition zu der zu stimulierenden Gehirnregion und eines Gehirnzustands des Benutzers, einschließlich wenigstens eines von Gehirnform des Benutzers, Gehirngröße und Kopfform, die unter Verwendung eines empfangenen MRT-Bildes des Benutzers erfasst werden.

2. Das System nach Anspruch 1, wobei das Magnetfeldänderungsmodul eine Magnetfeldstimulation durch Antreiben der Spule mit der Magnetfeldintensität durchführt, die durch Berechnen der bei dem vorbestimmten Verhältnis R1 gemessenen motorischen Schwelle des Benutzers auf Grundlage der zu stimulierenden Gehirnregion und des Gehirnzustands des Benutzers bestimmt wird.

3. Das System nach Anspruch 1, wobei der magnetfeldbezogene Parameter wenigstens eines von Frequenz, Zeit, Periode, Pulsbreite und Intensität des Magnetfelds umfasst.

4. Das System nach Anspruch 1, wobei das System ferner eine Gehirnnavigationseinrichtung aufweist, die eingerichtet ist zum Erfassen einer Position der zu stimulierenden Gehirnregion durch Analysieren des MRT-Bildes des Benutzers und zum Führen einer Position der Magnetfelderzeugungseinrichtung.

5. Das System nach Anspruch 4, wobei das System ferner Folgendes aufweist:
einen MRT-Datenspeicher, eingerichtet zum Empfangen und Speichern des MRT-Bildes des Benutzers und von Daten über die Position der zu stimulierenden Gehirnregion; und
ein Zuordnungsmodul, eingerichtet zum Erfassen eines tatsächlichen Kopfbildes des Benutzers in Echtzeit unter Verwendung einer Kamera und zum Zuordnen des in Echtzeit erfassten tatsächlichen Kopfbildes zu dem MRT-Bild des Gehirns des Benutzers, das die Daten über die Position der zu stimulierenden Gehirnregion anzeigt, die im MRT-Datenspeicher gespeichert sind.

6. Das System nach Anspruch 1, wobei das System ferner eine kognitive Trainingseinrichtung aufweist, die mit der Magnetfelderzeugungseinrichtung gekoppelt ist, um ein kognitives Training durch Präsentieren einer vorbestimmten Frage entsprechend der Gehirnregion durchzuführen, um eine durch die Magnetfelderzeugungseinrichtung stimulierte Gehirnregion zu aktivieren, nachdem das Gehirn durch die Magnetfelderzeugungseinrichtung stimuliert wurde.

7. Das System nach Anspruch 6, wobei das System ferner eine integrierte Steuereinheit aufweist, die mit der Magnetfelderzeugungseinrichtung und der kognitiven Trainingseinrichtung kommuniziert und die Magnetfelderzeugungseinrichtung und die kognitive Trainingseinrichtung gleichzeitig oder abwechselnd antreibt.

8. Das System nach Anspruch 7, wobei die integrierte Steuereinheit oder die kognitive Trainingseinrichtung ferner wenigstens einen Speicher aufweist und der Speicher eine in einem Cloud-Server konfigurierte Datenbank (DB) ist.

9. Das System nach Anspruch 5, ferner einen Roboterarm zum Empfangen von Koordinaten der Position der zu stimulierenden Gehirnregion in dem tatsächlichen Kopf aufweisend, die durch das Zuordnungsmodul erfasst wird, und zum Bewegen der Spule entsprechend den Positionskoordinaten; und
einen Laserscanner zum Scannen des Kopfes des Benutzers zum Erfassen einer Kopfposition, die der Position der zu stimulierenden Gehirnregion entspricht,
wobei der Laserscanner den tatsächlichen Kopf scannt, um Daten über eine Position des tatsächlichen Kopfes zu erfassen, und Daten über die Kopfposition an den Roboterarm überträgt.

## Revendications

1. Système de stimulation magnétique comprenant un dispositif de génération de champ magnétique, dans lequel le dispositif de génération de champ magnétique (100) inclut :
une unité de commande (110) configurée pour déterminer un champ magnétique correspondant en tenant compte de données d'utilisateur, incluant un seuil moteur et une région du cerveau devant être stimulée ;
un module électrique (120) connecté à une bobine (160) configurée pour générer un champ magnétique et alimentant en électricité la bobine pour générer le champ magnétique déterminé ; et
un capteur de mesure de seuil moteur, et
l'unité de commande inclut :
un module de mesure de seuil moteur (1102) configuré pour mesurer le seuil moteur d'un utilisateur en utilisant un module de changement de champ magnétique (1101) par l'ajustement par pas de l'intensité de champ magnétique par le module de changement de champ magnétique, et mesurer le seuil moteur d'utilisateur par l'augmentation de la fréquence de champ magnétique d'un rapport R1 prédéterminé et ensuite par l'ajustement par pas de l'intensité de champ magnétique à nouveau lorsque l'intensité de champ magnétique atteint la sortie de champ magnétique maximale, dans lequel la mesure du seuil moteur de l'utilisateur inclut :
un premier niveau de réalisation d'une stimulation de champ magnétique sur une région de cortex moteur de l'utilisateur par la bobine pour vérifier si un changement dans un mouvement du doigt ou un changement dans la motilité de l'utilisateur se produit à l'aide du capteur de mesure de seuil moteur ; un deuxième niveau d'augmentation de l'intensité de champ magnétique du rapport R1 prédéterminé jusqu'à ce qu'un changement dans le mouvement du doigt ou dans la motilité se produise lorsqu'aucun changement dans le mouvement du doigt ou lorsqu'aucun changement dans la motilité ne se produit ; et un troisième niveau d'augmentation de la fréquence de champ magnétique lorsque l'intensité de champ magnétique atteint une sortie de champ magnétique maximale prédéterminée, et de retour au deuxième niveau ; et
lorsque le changement dans le mouvement du doigt ou le changement dans la motilité se produit, le module de mesure de seuil moteur est configuré pour diminuer l'intensité de champ magnétique à un rapport R2 prédéterminé qui est inférieur à R1 en utilisant le module de changement de champ magnétique et délivrer en sortie l'intensité de champ magnétique diminuée, et lorsque le mouvement du doigt ou le changement dans la motilité ne se produit plus, le module de mesure de seuil moteur est configuré pour déterminer l'intensité de champ magnétique comme étant le seuil moteur ;
le module de changement de champ magnétique étant configuré pour commander le champ magnétique délivré en sortie par l'ajustement d'un paramètre relatif au champ magnétique, et
le module de changement de champ magnétique étant configuré pour déterminer la sortie de champ magnétique sur la base du seuil moteur mesuré, et une distance d'une position de traitement à la région du cerveau devant être stimulée, et un état du cerveau de l'utilisateur incluant au moins l'une parmi la forme du cerveau, la taille du cerveau et la forme de la tête de l'utilisateur, acquise à l'aide d'une image d'IRM reçue de l'utilisateur.

2. Système selon la revendication 1, dans lequel le module de changement de champ magnétique réalise une stimulation de champ magnétique par l'attaque de la bobine avec l'intensité de champ magnétique déterminée par le calcul du seuil moteur d'utilisateur mesuré au rapport R1 prédéterminé sur la base de la région du cerveau devant être stimulée et de l'état du cerveau de l'utilisateur.

3. Système selon la revendication 1, dans lequel le paramètre relatif au champ magnétique inclut au moins l'un parmi la fréquence, la durée, la période, la largeur d'impulsion et l'intensité du champ magnétique.

4. Système selon la revendication 1, comprenant en outre un dispositif de navigation dans le cerveau détectant une position de la région du cerveau devant être stimulée par l'analyse de l'image d'IRM de l'utilisateur et le guidage d'une position du dispositif de génération de champ magnétique.

5. Système selon la revendication 4, comprenant en outre :
une mémoire de données d'IRM configurée pour recevoir et stocker l'image d'IRM de l'utilisateur et des données sur la position de la région du cerveau devant être stimulée ; et
un module de cartographie configuré pour capturer une image réelle de la tête de l'utilisateur en temps réel en utilisant un appareil de prise de vues, et cartographiant l'image réelle de la tête capturée en temps réel avec l'image d'IRM du cerveau de l'utilisateur affichant les données sur la position de la région du cerveau devant être stimulée qui est stockée dans la mémoire de données d'IRM.

6. Système selon la revendication 1, comprenant en outre un dispositif d'entraînement cognitif lié au dispositif de génération de champ magnétique pour réaliser un entraînement cognitif par la présentation d'une question prédéterminée correspondant à la région du cerveau pour activer une région du cerveau stimulée par le dispositif de génération de champ magnétique après que le cerveau est stimulé par le dispositif de génération de champ magnétique.

7. Système selon la revendication 6, comprenant en outre un opérateur intégré communiquant avec le dispositif de génération de champ magnétique et le dispositif d'entraînement cognitif, et entraînant simultanément ou en alternance le dispositif de génération de champ magnétique et le dispositif d'entraînement cognitif.

8. Système selon la revendication 7, dans lequel l'opérateur intégré ou le dispositif d'entraînement cognitif inclut en outre au moins une mémoire, et la mémoire est une base de données (DB) configurée dans un serveur en nuage.

9. Système selon la revendication 5, comprenant en outre un bras robotisé recevant des coordonnées de la position de la région du cerveau devant être stimulée dans la tête réelle qui est acquise par le module de cartographie et déplaçant la bobine pour qu'elle corresponde aux coordonnées de position ; et
un dispositif de balayage par laser balayant la tête de l'utilisateur pour détecter une position de tête correspondant à la position de la région du cerveau devant être stimulée,
dans lequel le dispositif de balayage par laser balaie la tête réelle pour acquérir des données sur une position de la tête réelle, et émet des données sur la position de la tête vers le bras robotisé.
